# EUROPEAN PATENT APPLICATION

(11) **EP 4 403 626 A1**
(43) Date of publication of application: **24.07.2024**
(21) Application number: 21957266.6
(22) Date of filing: 26.10.2021
(51) Int. Cl.: C12N 5/09, C12N 5/071, C12N 5/074, C12Q 1/02

(54) **CULTURE MEDIUM AND CULTURE METHOD FOR LUNG CANCER EPITHELIAL CELLS, AND APPLICATION THEREOF**

(30) Priority: 15.09.2021 CN 202111079965
(71) Applicant: Precedo Pharmaceuticals Co., Ltd., Hefei, Anhui 230094 (CN)
(72) Inventor: LIU, Qingsong, Hefei, Anhui 230031 (CN); HU, Jie, Hefei, Anhui 230031 (CN); CHEN, Cheng, Hefei, Anhui 230031 (CN); HUANG, Tao, Hefei, Anhui 230031 (CN)
(74) Representative: Abel & Imray LLP
(86) International application number: PCT/CN2021/126229
(87) International publication number: WO 2023/039999

(57) **Abstract**

A primary cell culture medium for culturing primary lung cancer epithelial cells, a culture method using the primary cell culture medium, and an application thereof in drug efficacy evaluation and screening. The culture medium contains an MST1/2 kinase inhibitor, a ROCK kinase inhibitor, a fibroblast growth factor, at least one additive selected from a B27 additive and an N2 additive, an epidermal growth factor, transferrin, gastrin, and a TGFβ type I receptor inhibitor.

## Description

### Technical Field

The invention relates to the field of medical technology, in particular to a culture medium and a culture method for culturing or expanding primary lung cancer epithelial cells *in vitro,* and also to a method and application of the cultured cells in the efficacy evaluating and screening of drugs.

### Background of the Invention

Lung cancer is currently the most common respiratory tract tumor in the world. There are more than 1.8 million new patients of lung cancer worldwide every year. As the most common malignant tumor in clinical practice, lung cancer is mainly treated by surgery, chemotherapy, radiotherapy, molecular targeted therapy and immunotherapy, among which surgery, chemotherapy and radiotherapy are the most commonly used means. However, there are limitations in the suitable population for these clinical treatments. In recent years, with the rise and development of molecular biology, pharmacotherapy of tumor has shown a diversified trend, among which molecular targeted drugs have become a hot research topic in clinical treatment of lung cancer due to their strong targeting and high safety. However, among the many clinical treatment options, it is particularly important to choose the one that suits the patient. Although genetic testing is used as an indicator, for some patients who have no gene mutation, or for some patients who have a certain mutation but there are multiple targeted drugs for the mutation, it may be difficult to determine a treatment plan in clinical practice. In addition to gene sequencing, primary cell culture of samples from lung cancer patients *in vitro* has become an important means for predicting efficacy *in vitro* and guiding clinical medication in the future. However, rapid acquisition of primary lung cancer cells *in vitro* has always been an urgent technical problem to be solved.

Functional testing refers to the method of detecting the sensitivity of anti-tumor drugs on cells of cancer patient *in vitro.* The key to apply this method is to develop tumor cell models that have short growth cycle and can represent the biological characteristics of patients with lung cancer. In addition, the cell model should be easy to operate to quickly and efficiently predict the efficacy of clinical medication, so as to give precise medication guidance to cancer patients in a timely manner. However, the normally low success rates and long growth cycles of *in vitro* establishment of cell models from the primary tumor cells of cancer patients, and the problems such as excessive proliferation of mesenchymal cells (such as fibroblasts, and the like), all restrict the development in this field. There are currently two techniques for culturing primary epithelial / stem cells that are relatively mature in the field of functional testing of tumor cells. One is the technique that uses irradiated feeder cells and ROCK inhibitor Y27632 to promote the growth of primary epithelial cells to investigate the drug sensitivity of individual patients, i.e. the cell conditional reprogramming technique (Liu et al., Am J Pathol, 180: 599-607, 2012). Another technique is 3D culture of adult stem cells *in vitro* to obtain organoids similar to tissues and organs (Hans Clevers et al., Cell, 11; 172(1-2): 373-386, 2018).

However, both techniques have certain limitations. Cell reprogramming is a technique in which the autologous primary epithelial cells from a patient are cultured with mouse-derived feeder cells. When the primary cells from a patient are tested for drug sensitivity, the presence of these mouse-derived cells may interfere with the drug sensitivity testing results of the patient's autologous primary cells; however, if the mouse-derived feeder cells are removed, the patient's autologous primary cells will detach from the reprogramming environment, and the cell proliferation rate and intracellular signaling pathways will undergo significant changes (Liu et al., Am J Pathol, 183(6): 1862-1870, 2013; Liu et al., Cell Death Dis., 9(7) : 750, 2018), thereby greatly affecting the response of the patient's autologous primary cells to the drug. Organoid is a technique that embeds the patient's autologous primary epithelial cells in the extracellular matrix for three-dimensional culture *in vitro,* and there is no interference problem from mouse-derived feeder cells because the feeder cells are not required in this technique. However, the culture medium in the organoid technique needs to be added with a variety of specific growth factors (such as Wnt proteins and R-spondin family proteins), which is expensive and unsuitable for extensive use in clinical. In addition, the organoid needs to be embedded in the extracellular matrix gel during the entire culture process, and the plating steps of cell inoculation, passage, and drug sensitivity test are cumbersome and time-consuming as compared with 2D culture operations. Additionally, the size of the organoid formed by this technique is difficult to control, and some organoids may grow too large and cause an internal necrosis. Therefore, the organoid technique has poorer operability and applicability than the 2D culture technique. It requires professional technicians to operate, and thus, is not suitable for extensive and wide use in clinical for *in vitro* drug sensitivity testing (Nick Barker, Nat Cell Biol, 18(3): 246-54, 2016).

In view of the limitations of the above techniques, it is necessary to develop a culture technique for primary lung cancer epithelial cells in clinic, which may provide short culture period, controllable cost, and convenient operation with no interference from exogenous cells. When this technique is applied to construct a primary tumor cell model of lung cancer, the cultured lung cancer cells can represent the biological characteristics of the lung cancer patients themselves. By evaluating *in vitro* the sensitivity of anti-tumor drugs in the cell models derived from individual cancer patients, the response rate of the anti-tumor drug can be improved in clinic, and the pains caused by inappropriate drugs to the patients and the waste of medical resources can be reduced.

### Summary of the Invention

In view of the deficiencies of the prior art, the invention intends to provide a culture medium for culturing primary lung cancer epithelial cells and a method for culturing primary lung cancer epithelial cells using the culture medium. The culture medium and the culture method for primary lung cancer epithelial cells of the invention can achieve the goal of short *in vitro* culture period, controllable cost, and convenient operation with no interference from exogenous cells. When the technique is applied to construct a primary tumor cell model of lung cancer, the primary lung cancer cells with the biological characteristics of the lung cancer patients can be obtained, which can be applied in new drug screening and *in vitro* drug sensitivity test.

One aspect of the invention is to provide a primary cell culture medium for culturing primary lung cancer epithelial cells, comprising an MST1/2 kinase inhibitor, a ROCK kinase inhibitor selected from at least one of Y27632, Fasudil, and H-1152; a fibroblast growth factor; at least one additive selected from B27 additive and N2 additive; an epidermal growth factor; transferrin; gastrin; and a TGFβ type I receptor inhibitor selected from at least one of A83-01, SB431542, Repsox, SB505124, SB525334, SD208, LY36494, and SJN2511, wherein the MST1/2 kinase inhibitor comprises a compound of Formula (I) or a pharmaceutically acceptable salt, or a solvate thereof, 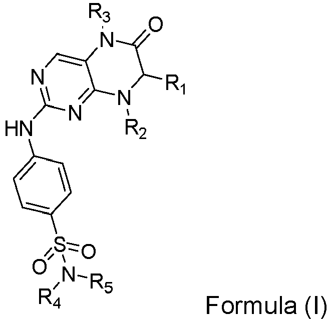 Wherein,
R₁ is selected from C1-C6 alkyl, C3-C6 cycloalkyl, C4-C8 cycloalkylalkyl, C2-C6 spirocycloalkyl, and aryl (e.g., phenyl and naphthyl, etc.) optionally substituted with 1-2 independent R₆, aryl C1-C6 alkyl (e.g., phenylmethyl, etc.) optionally substituted with 1-2 independent R₆ and heteroaryl (e.g., thienyl, etc.) optionally substituted with 1-2 independent R₆;
R₂ and R₃ are each independently selected from C1-C6 alkyl, preferably C1-C3 alkyl, more preferably methyl;
R₄ and R₅ are each independently selected from hydrogen, C1-C6 alkyl, C3-C6 cycloalkyl, C4-C8 cycloalkylalkyl, hydroxyl C1-C6 alkyl, C1-C6 haloalkyl, C1-C6 alkylamino C1-C6 alkyl, C1-C6 alkoxy C1-C6 alkyl, and C3-C6 heterocyclyl C1-C6 alkyl (the heterocyclyl is selected from e.g., piperidyl, tetrahydropyranyl, etc.);
R₆ is selected from halogen (preferably fluoro and chloro, more preferably fluoro), C1-C6 alkyl (preferably methyl), C1-C6 alkoxy (preferably methoxy), and C1-C6 haloalkyl (preferably trifluoromethyl).

In a preferable embodiment, the MST1/2 kinase inhibitor comprises a compound of Formula (Ia) or a pharmaceutically acceptable salt, or a solvate thereof, 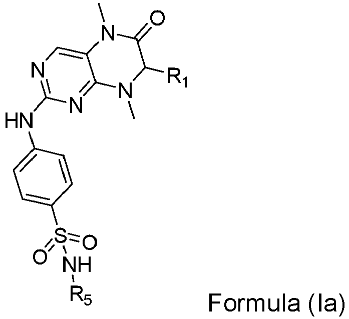 Wherein,
R₁ is selected from C1-C6 alkyl, phenyl optionally substituted with 1-2 independent R₆, thienyl optionally substituted with 1-2 independent R₆, and phenylmethyl optionally substituted with 1-2 independent R₆; more preferably, R₁ is phenyl optionally substituted with 1-2 independent R₆;
R₅ is selected from hydrogen, C1-C6 alkyl, and C3-C6 cycloalkyl; more preferably, R₅ is hydrogen;
R₆ is independently selected from halogen, C1-C6 alkyl, and C1-C6 haloalkyl; more preferably, R₆ is fluoro, methyl or trifluoromethyl.

Preferably, the MST1/2 kinase inhibitor is at least one selected from the following compounds or a pharmaceutically acceptable salt, or a solvate thereof.

| | | | |
|---|---|---|---|
| 1 | 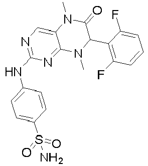 | 2 | 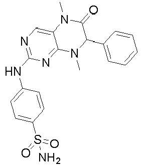 |
| 3 | 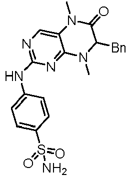 | 4 | 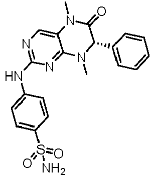 |
| 5 | 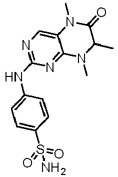 | 6 | 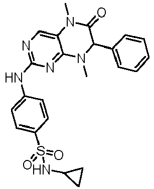 |
| 7 | 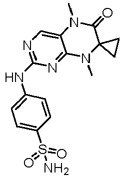 | 8 | 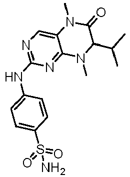 |
| 9 | 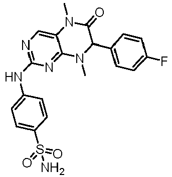 | 10 | 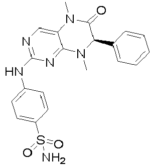 |
| 11 | 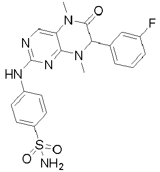 | 12 | 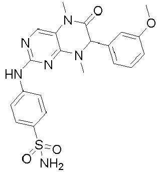 |
| 13 | 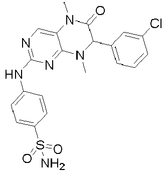 | 14 | 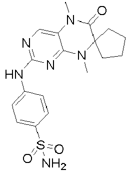 |
| 15 | 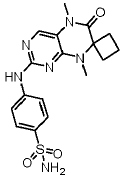 | 16 | 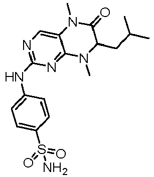 |
| 17 | 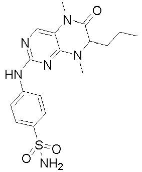 | 18 | 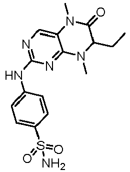 |
| 19 | 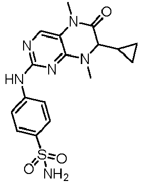 | 20 | 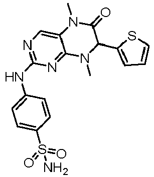 |
| 21 | 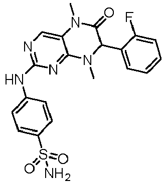 | 22 | 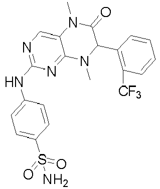 |
| 23 | 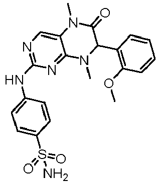 | 24 | 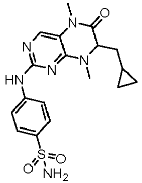 |
| 25 | 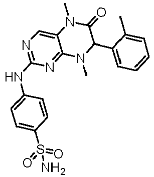 | 26 | 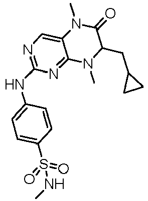 |
| 27 | 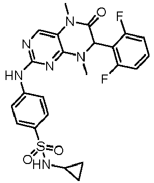 | 28 | 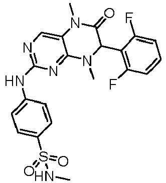 |
| 29 | 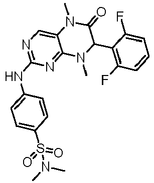 | 30 | 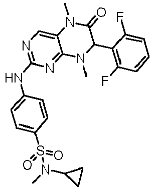 |
| 31 | 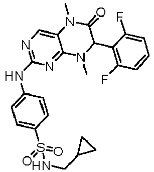 | 32 | 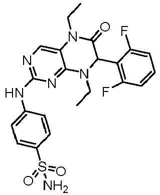 |
| 33 | 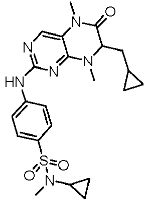 | 34 | 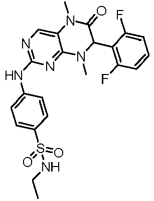 |
| 35 | 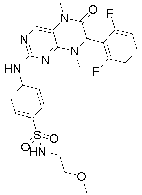 | 36 | 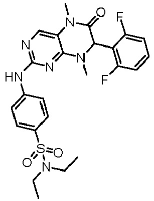 |
| 37 | 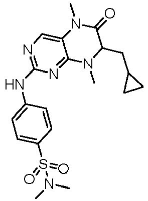 | 38 | 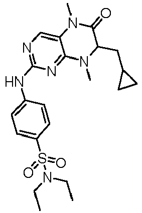 |
| 39 | 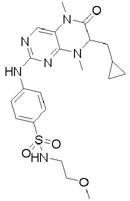 | 40 | 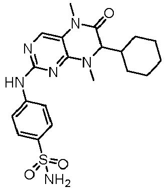 |
| 41 | 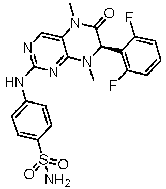 | 42 | 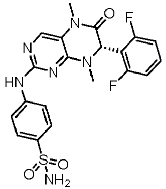 |
| 43 | 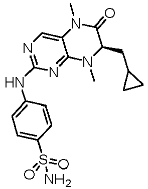 | 44 | 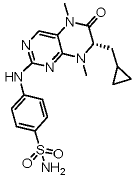 |
| 45 | 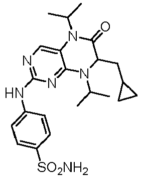 | 46 | 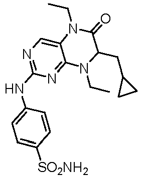 |
| 47 | 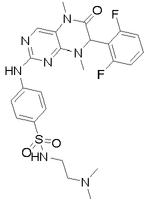 | 48 | 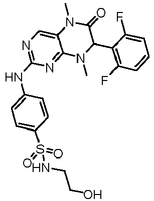 |
| 49 | 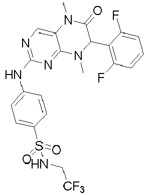 | 50 | 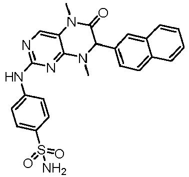 |
| 51 | 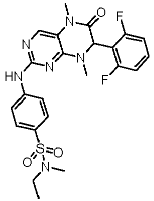 | 52 | 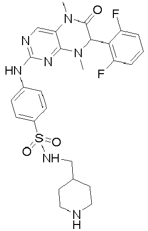 |
| 53 | 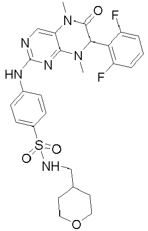 | 54 | 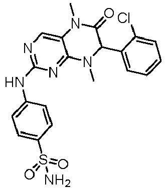 |
| 55 | 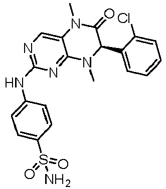 | 56 | 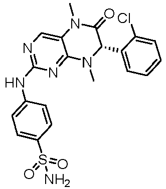 |
| 57 | 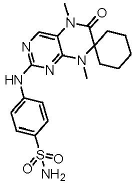 | 58 | 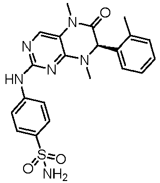 |
| 59 | 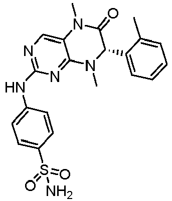 | | |

Most preferably, the MST1/2 kinase inhibitor of the invention is Compound 1.

In the embodiment of the invention, the amount of the MST1/2 kinase inhibitor in the culture medium is usually 2.5 µM-15 µM, preferably 2.5 µM-10 µM.

In yet another embodiment, the ROCK kinase inhibitor is preferably Y27632. In addition, preferably, the amount of the ROCK kinase inhibitor in the culture medium is usually 2.5 µM-18 µM, preferably 5 µM-15 µM.

In a preferred embodiment, the amount of the fibroblast growth factor is 2.5-80 ng/ml, preferably 5-40 ng/ml; the volume concentration of the B27 additive or the N2 additive in the culture medium is 1 :25-1 :800, preferably 1:25-1:200; the amount of the epidermal growth factor is 2.5-80 ng/ml, preferably 10-40 ng/ml; the amount of transferrin is 2.5-80 ng/ml, preferably 5-80 ng/ml; the amount of gastrin is 2.5-80 ng/ml, preferably 5-40 ng/ml; the amount of the TGFβ type I receptor inhibitor is 62.5-800 nM, preferably 125-500 nM.

The medium formulation of the invention also contains an initial medium selected from the group consisting of DMEM/F12, DMEM, F12 or RPMI-1640; and one or more antibiotics selected from the group consisting of streptomycin / penicillin, amphotericin B and Primocin. In some embodiments, the initial medium is preferably DMEM/F12, and the antibiotic is preferably Primocin. In a further preferred embodiment, the amount of Primocin in the culture medium is 25 µg/ml-400 µg/ml, preferably 50 µg/ml-200 µg/ml.

Compared with the composition of the medium for cell conditional reprogramming and the composition of the medium for organoid of lung cancer epithelial cells, the composition of the medium of the invention is supplemented with a MST1/2 kinase inhibitor, but does not contain uncertain components such as serum, bovine pituitary extract or the like, niche factors that are necessary for culture of organoid such as Wnt agonists, R-spondin family proteins, BMP inhibitors or the like, nor does it contain nicotinamide or N-acetylcysteine, thereby greatly reducing the cost of the medium, simplifying the operation process of preparing the medium, and realizing the *in vitro* culture of the primary lung cancer epithelial cells with controllable cost and convenient operation.

In the invention, the primary lung cancer epithelial cells can be lung cancer cells, normal lung cancer epithelial cells, or lung cancer epithelial stem cells.

One aspect of the invention is to provide a method for culturing the primary lung cancer epithelial cells, comprising the following steps:
(1) Preparing the primary cell culture medium of the invention according to the above formulation.
(2) Coating a culture vessel with extracellular matrix gel dilution.

Specifically, as the extracellular matrix gel, a low growth factor type extracellular matrix gel can be used, for example, commercially available Matrigel (purchased from Corning) or BME (purchased from Trevigen) can be used. More specifically, the extracellular matrix gel is diluted with a serum-free culture medium, which may be DMEM/F12 (purchased from Corning). The dilution ratio of the extracellular matrix gel is 1:50-1:400, preferably 1:100-1:200. The coating method comprises adding the diluted extracellular matrix gel into the culture vessel to cover the bottom of the culture vessel completely, and standing for 30 minutes or more, preferably standing and coating at 37°C, preferably for a coating time of 30-60 minutes. After the coating is completed, the excess extracellular matrix gel diluent is discarded and the culture vessel is ready for later use.

### (3) Isolating the primary lung cancer epithelial cells from the lung cancer tissue.

The primary lung cancer epithelial cells can be derived, for example, from the lung cancer surgery samples and biopsy samples. For example, the lung cancer tissue samples are derived from the cancer tissue samples by surgical resection from lung cancer patients who have given informed consent, and the biopsy samples are collected from intrapulmonary lesion under ultrasound guidance. Collection of the aforementioned tissue samples is performed within half an hour of a patient's surgical excision or biopsy. More specifically, in a sterile environment, the tissue sample from non-necrotic sites is cut, with its volume of 5 mm³ or more, and then the tissue sample is placed in a pre-cooled tissue transport fluid, which is contained in a plastic sterile centrifuge tube with a lid, and transported to the laboratory on ice. Wherein, the tissue transport fluid contains DMEM/F12, the MST1/2 kinase inhibitor (e.g., Compound 1) of the invention and 0.2-0.4 vol% of Primocin. The concentration range of the MST1/2 kinase inhibitor of the invention is 0.3-10 µM, preferably 2-5 µM, and more preferably 3 µM; and the concentration range of Primocin is 25-400 µg/ml, preferably 50-200 µg/ml, more preferably 100 µg/ml.

In a biological safety cabinet, the tissue sample is transferred to a cell culture dish, which is then rinsed with the tissue transport fluid, and the blood cells on the surface of the tissue sample are washed away. The rinsed tissue sample is transferred to another new culture dish, with the addition of 1-3 ml of the tissue transport fluid, and the tissue sample is divided into tissue fragments less than 3 mm³ in volume using a sterile scalpel blade and a forceps.

The tissue sample fragments are transferred to a centrifuge tube, which is centrifuged at 1000-3000 rpm for 3-5 minutes in a tabletop centrifuge (Sigma, 3-18K); after discarding the supernatant, the tissue transport fluid and the tissue digestive solution are added in a ratio of 1:1 (the dosage is about 5 ml of tissue digestive solution per 10 mg of tissue; the preparation method of the tissue digestive solution comprises: dissolving 1-2 mg/ml collagenase II, 1-2 mg/ml collagenase IV, 50-100 U/ml deoxyribonucleic acid I, 0.5-1 mg/ml hyaluronidase, 0.1-0.5 mg/ml calcium chloride, 5-10 mg/ml bovine serum albumin in HBSS and RPMI-1640 with a volume ratio of 1:1); then the sample is numbered and sealed with sealing film, and is then digested in a constant-temperature shaker (Zhichu Instrument ZQLY-180N) at 37°C, 200-300 revolutions; whether the digestion is completed is determined via observation every 1 hour; if no obvious tissue block is found, the digestion can be terminated; otherwise, it is continued for digestion until the digestion is sufficient, with the digestion time ranges from 4 to 8 hours. After digestion, undigested tissue blocks are filtered through a cell filter screen (the cell screen mesh size is, for example 70 µm); the tissue blocks on the filter screen are rinsed with the tissue transport fluid; the residual cells are rinsed into a centrifuge tube and centrifuged with a tabletop centrifuge at 1000-3000 rpm for 3-5 minutes. After discarding the supernatant, the remaining cell pellet is observed to determine whether blood cells are remained; if there are blood cells, 3-5 ml blood cell lysate (purchased from Sigma) is added, which is then mixed well, lysed at 4°C for 10-20 minutes, with shaking and mixing well once every 5 minutes; after lysis, the resultant is take out and centrifuged at 1000-3000 rpm for 3-5 minutes. After discarding the supernatant, the primary cell culture medium of the invention is added for resuspension. The total number of cells is obtained by counting with a flow imaging counter (JIMBIO FIL, Jiangsu Jimbio Technology Co., Ltd.).

### (4) Inoculating the primary lung cancer epithelial cells isolated in step (3) in the coated culture vessel, and culturing by using the primary cell culture medium obtained in step (1).

More specifically, primary lung cancer cells are inoculated in one well of a multi-well plate at a density of 2×10⁴ to 8×10⁴ cells/cm² (e.g., 4 × 10⁴ cells/cm²); 2-3 ml of primary epithelial cell culture medium is added, and then the plate is cultured in a cell incubator for 8-16 days, for example, under the conditions of 37°C, 5% CO₂; fresh primary cell culture medium is used for replacing every 4 days during the culture; digestion and passaging are performed when the primary lung cancer epithelial cells grow to a cell density that accounts for about 80% to 90% of the bottom area of the multi-well plate.

This inoculation step does not require the use of feeder cells, and thus eliminates the steps of culturing and irradiating feeder cells compared with the cell conditional reprogramming technique. Compared with the organoid technique, this step does not need to mix the primary cells with the matrix gel uniformly on ice to form gel droplets and wait for the solidification of the gel droplets before adding the medium, either, and thus the pre-coated culture vessel can be directly used for inoculation of primary cells. In addition, compared with the organoid technique, the coated culture vessel only requires a small amount of diluted extracellular matrix gel, which saves the amount of the expensive extracellular matrix gel and also simplifies the operation steps.

Optionally, after culturing the inoculated primary lung cancer epithelial cells for 8-16 days, when the cell clones formed in the culture container converge to cover 80% of the bottom area, the supernatant is discarded, and 0.5-2 ml of 0.05% trypsin (purchased from Thermo Fisher) is added for cell digestion, which is then incubated at room temperature for 5-20 minutes. The digested cells are resuspended in 1-4 ml of DMEM/F12 culture liquid containing, for example, 5% (v/v) fetal bovine serum, 100 U/ml penicillin, and 100 µg/ml streptomycin, and are centrifuged at 1000-3000 rpm for 3-5 minutes. The digested single cells are resuspended using the primary cell culture medium of the invention, and the obtained cell suspension is placed in a T25 cell culture flask coated with extracellular matrix gel for continuous culture. The coating step of T25 cell culture flask is the same as that in step (2).

The expanded lung cancer epithelial cells grow in 2D, avoiding the non-uniform size of organoids and internal necrosis in overgrown organoids that may occur in the expansion using organoid technique.

In another aspect, the lung cancer epithelial cells, especially the lung cancer tumor cells, cultured by the culturing method for primary lung cancer epithelial cells of the invention, can be used for efficacy evaluating and screening of drugs, which comprises the following steps:
(1) Obtaining the primary lung cancer epithelial cells, more preferably, obtaining the cancer tissue samples or the biopsy cancer tissue samples derived from the lung cancer patients; isolating the primary lung cancer epithelial cells, and culturing and expanding the primary lung cancer epithelial cells (particularly the primary lung cancer cells) according to the method for culturing the primary lung cancer epithelial cells described above to a cell number of at least the magnitudes of 10⁵, preferably at least the magnitudes of 10⁶.
(2) Selecting the drug to be tested.
(3) Based on the maximum plasma concentration Cₘₐₓ of the drug as a reference, taking 2-5 times of Cₘₐₓ as the initial concentration, and diluting the drug into different drug concentration gradients, such as 5-10 drug concentration gradients, preferably 6-8 drug concentration gradients.
(4) Digesting the lung cancer epithelium cells cultured in step (1) into a single cell suspension, counting the cell number with a flow imaging counter, diluting the single cell suspension with the primary cell culture medium of the invention, adding the diluted cell suspension evenly to the multi-well plate at a density of 2000-4000 cells per well, e.g., 50 µL of cell dilution per well, and adhering overnight.

This step avoids the problem of the cell reprogramming technique that the presence of feeder cells may interfere with the primary cell counting and the subsequent primary cell viability assay, and eliminates the need of the tedious step of mixing, embedding and then plating the cell suspension with matrix gel on ice as that in the organoid technique, which greatly simplifies the operation process and enhances the operability and practicality of the technique. Since the inoculated cells are single-cell suspensions rather than 3D structures like organoid, this technique may result in a more uniform number of plating cells and less variation in cell numbers between wells when compared with the organoid technique, making it more suitable for subsequent high-throughput drug screening operations.

(5) Adding the selected candidate drugs such as traditional chemotherapeutic drugs, targeted drugs, antibody drugs, or combination thereof with gradient dilutions, to the adherent cells obtained in step (4), using a high-throughput automated workstation.

(6) Several hours after drug addtion, for example, 72 hours after drug addition, Cell-Titer Glo Luminescent Cell Viability Assay Kit (purchased from Promega) is used for detection of the survival rate of the lung cancer epithelial cells to screen for drug activity.

Specifically, each well is added with, for example, 10 µL of Cell Titer-Glo reagent (purchased from Promega), and after uniformly shaking, the chemiluminescence intensity is measured with a fluorescence microplate reader for each well. Taking the drug concentration as the abscissa and the fluorescence intensity as the ordinate, GraphPad Prism software is used to draw the drug dose-effect curve based on the measured values, and the inhibitory intensity of the drugs on the proliferation of the tested cells are calculated.

In the application of primary lung cancer cells of the invention in drug screening and *in vitro* drug sensitivity detection, the feeder cells will not interfere with the detection results as in the cell reprogramming technique because a cell co-culture system is not used. In addition, due to the 2D growth of the cells, the interaction time with drug is shorter than the time of drug detection in the organoid technique (the average administration time in the organoid technique is 6 days).

The beneficial effects of the invention also include:
(1) the success rate for culturing the primary lung cancer epithelial cell is improved, with a success rate of 80% or more;
(2) the lung cancer epithelial cells primary cultured *in vitro* is ensured to reproduce the pathological phenotype and the heterogeneity of the owner patient of the primary cells;
(3) the cultured primary lung cancer epithelial cells are not interfered by fibroblasts, and purified lung cancer epithelial cells can be obtained;
(4) the composition of the medium does not contain serum, and thus, it is not affected by the quality and the quantity of the serum from different batches;
(5) the lung cancer epithelial cells are expanded with high efficiency, with a magnitude of 10⁶ of lung cancer epithelial cells being successfully expanded within about two weeks from the starting of 10⁴-level cell count, and the expanded lung cancer epithelial cells have the ability of continuous passage;
(6) there is no need to operate on ice and to dissociate the matrix gel in the passage step, and the digestion and passage of cells can be completed within 10-15 minutes;
(7) the cost for culture is controllable, as the primary lung cancer culture medium does not require expensive Wnt agonists, R-spondin family proteins, BMP inhibitors and the like factors, which is a simplification and improvement of the existing culture medium for primary lung cancer epithelial cell organoid; inoculation of the cells does not need to use a higher concentration of extracellular matrix mixed to form gel droplets with primary cells, either, but only needs to use a small amount of dilution prepared from extracellular matrix gel, which saves the amount of higher-cost extracellular matrix;
(8) the operations are convenient: compared with the conditional reprogramming technique, this technique does not need to cultivate and irradiate the feeder cells, and thus avoiding the problem that the quality and quantity of feeder cells from different batches may affect the efficiency of primary cell culture; and the objects of plating and testing in the drug screen are only primary lung cancer epithelial cells, without interference of feeder cells as in the co-culture system required in the cell conditional reprogramming technique; compared with the organoid technique, in the method for coating extracellular matrix gel adopted in the invention, the culture vessels can be prepared in advance, and there is no need to embed cells in the matrix gel as in the organoid technique, and thus the operation steps are simple and easy;
(9) this technique can culture and provide the lung cancer epithelial cells with large quantity and high uniformity, which is suitable for high-throughput screening of new candidate compounds and high-throughput drug sensitivity functional tests *in vitro* for patients.

Using the cell culture medium of this embodiment, the lung cancer epithelial cells derived from humans or other mammals, including lung cancer cells, normal lung epithelial cells, lung cancer epithelial stem cells, or tissues comprising at least any of these cells, can be cultured. At the same time, the culture medium of the invention can also be used to develop a kit for expansion and culture of primary lung cancer cells *in vitro.*

Furthermore, the cells obtained by the culture method of this embodiment can be used in regenerative medicine, basic medical research of lung cancer epithelial cells, screening of drug responses, and development of new drugs for lung cancer, and the like.

### Description of Drawings

Figures 1A-1D show the effects of different factors in the culture medium on the proliferation of primary lung cancer cells.
Figure 2 shows the effect of increasing factors in the culture medium on the proliferation of primary lung cancer cells.
Figures 3A-3H show the effect of the concentration of each factor on the proliferation of primary lung cancer cells.
Figures 4A and 4B are photographs of lung cancer cells taken under an inverted microscope, which were cultured from the cells isolated from one clinical lung cancer tissue sample, by using the culture medium FLM of the invention, to Day 4 and Day 12, respectively.
Figures 5A-5D are photos taken under an inverte microscope after 12 days of culture under the conditions of 4 different culture mediums from the cells isolated from one surgically resected sample of lung cancer.
Figure 6 is a comparative diagram of cell proliferation effects obtained by culturing the cells isolated from ten surgically resected samples of lung cancer for 14 days under the conditions of 4 different culture mediums.
Figure 7 is a comparative graph of cell growth curves obtained by culturing the cells isolated from one clinical lung cancer tissue sample under the conditions of 4 different culture mediums.
Figure 8 shows comparison of immunohistochemical result between one surgically resected sample of lung cancer and that of lung cancer cells obtained by culturing the cells isolated from the sample using the culture medium FLM of the invention.
Figure 9 is a comparative graph of cell growth curves obtained by culturing the cells isolated from one clinical lung cancer tissue sample, under the conditions of the culture medium FLM of the invention and the culture mediums obtained by subtracting different components from FLM, respectively.
Figure 10 shows the analysis results of signal pathways related to cell proliferation after culturing cells using the culture medium FLM and the culture medium obtained by subtracting Compound 1 from FLM, respectively.
Figures 11A and 11B show the dose-response curves of primary lung cancer cells on different chemotherapeutic drugs and targeted drugs, wherein the primary lung cancer cells were obtained by culturing the surgically resected cancer tissue samples from two different lung cancer patients with the culture medium FLM of the invention, respectively.

### Detailed Description of the Invention

In the specification, the epithelial cells include differentiated epithelial cells and epithelial stem cells obtained from epithelial tissues. "Epithelial stem cells" refers to the cells having the ability of long-term self-renewal and to differentiate towards epithelial cells, and to the stem cells which are originated from epithelial tissues. Examples of epithelial tissues include cornea, oral mucosa, skin, conjunctiva, bladder, renal tubule, kidney, digestive organs (esophagus, stomach, duodenum, small intestine (including jejunum and ileum), large intestine (including colon)), liver, pancreas, mammary gland, salivary gland, lacrimal gland, prostate, hair root, trachea, lung, etc. The cell culture medium of the embodiment is preferably the culture medium for culturing lung originated epithelial cells.

In addition, in this specification, "epithelial tumor cells" refers to the cells obtained by tumorigenesis of cells originated from the aforementioned epithelial tissues.

In the specification, "organoid" refers to a three-dimensional, organ-like cellular tissue formed by spontaneously organizing and aggregating cells in high density within a controlled space.

### [Preparation Examples of MST1/2 Kinase Inhibitors]

In the specification, MST1/2 kinase inhibitor refers to any inhibitor that directly or indirectly negatively regulates MST1/2 signaling. Generally, MST1/2 kinase inhibitors reduce the activity of MST1/2 kinase by, for example, binding to the same. Since MST1 and MST2 have similar structures, MST1/2 kinase inhibitors may be, for example, compounds that bind to MST1 or MST2 and reduce the activity thereof.

### 1. Preparation of MST1/2 kinase inhibitor Compound 1

### 4-((7-(2,6-Difluorophenyl)-5,8-dimethyl-6-oxo-5,6,7,8-tetrahydropteridin-2-yl) amino)benzsulfamide 1

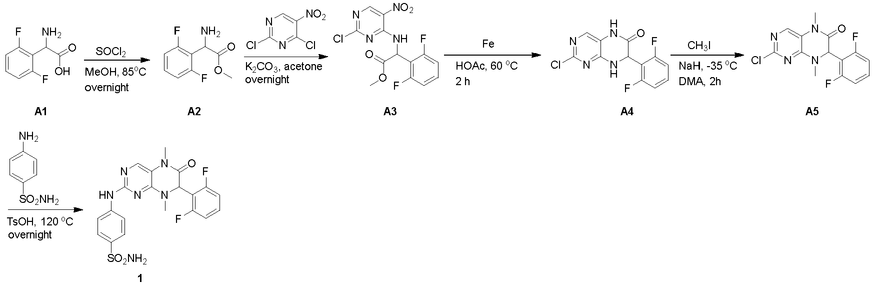

Methyl 2-amino-2-(2,6-difluorophenyl)acetate (A2): 2-amino-2-(2,6-difluorophenyl)acetic acid (2.0 g) and then methanol (30 ml) were added into a round bottom flask, followed by addition of thionyl chloride (1.2 ml) dropwise under an ice bath. The reaction system was reacted overnight at 85°C. After the completion of the reaction, the system was evaporated under reduced pressure to dry the solvent, and the obtained white solid was directly used in the next step.

Methyl 2-((2-chloro-5-nitropyrimidin-4-yl)amino)-2-(2,6-difluorophenyl) acetate (A3): methyl 2-amino-2-(2,6-difluorophenyl)acetate (2 g) and then acetone (30 ml) and potassium carbonate (2.2 g) were added into a round bottom flask, and then the system was cooled to -10°C with an ice salt bath, and then a solution of 2,4-dichloro-5-nitropyrimidine (3.1 g) in acetone was slowly added. The reaction system was stirred overnight at room temperature. After the completion of the reaction, the reaction mixture was filtered, the solvent was removed from the filtrate under reduced pressure, and the residue was purified by pressurized silica gel column chromatography to obtain compound A3. LC/MS: M+H 359.0.

2-Chloro-7-(2,6-difluorophenyl)-7,8-dihydropteridin-6(5H)-one (A4): methyl 2-((2-chloro-5-nitropyrimidin-4-yl)amino)-2-(2,6-difluorophenyl)acetate (2.5 g) and then acetic acid (50 ml) and iron powder (3.9 g) were added into a round bottom flask. The reaction system was stirred at 60°C for two hours. After the completion of the reaction, the reaction system was evaporated under reduced pressure to dry the solvent, and the resultant was neutralized to alkaline with saturated sodium bicarbonate solution and was extracted with ethyl acetate. The organic phase was washed with water and saturated brine and dried with anhydrous sodium sulfate. The organic phase was filtered and evaporated to dryness under reduced pressure to obtain a crude product. The crude product was washed with diethyl ether to obtain compound A4. LC/MS: M+H 297.0.

2-Chloro-7-(2,6-difluorophenyl)-5,8-dimethyl-7,8-dihydropteridin-6(5H)-one (A5): 2-chloro-7-(2,6-difluorophenyl)-7,8-dihydropteridin-6(5H)-one (2 g) and N,N-dimethylacetamide (10 ml) were added into a round bottom flask, and cooled to -35°C, followed by addition of iodomethane (0.9 ml) and then sodium hydride (615 mg), and the reaction system was stirred for two hours. After the completion of the reaction, the reaction mixture was quenched with water, and extracted with ethyl acetate. The organic phase was washed with water and saturated brine, respectively, and dried with anhydrous sodium sulfate. The organic phase was filtered and evaporated to dryness under reduced pressure to obtain a crude product. The crude product was washed with diethyl ether to obtain compound A5. LC/MS: M+H 325.0.

4-((7-(2,6-Difluorophenyl)-5,8-dimethyl-6-oxo-5,6,7,8-tetrahydropteridin-2-yl )amino)benzsulfamide (1): 2-chloro-7-(2,6-difluorophenyl)-5,8-dimethyl-7,8-dihydropteridin-6(5H)-one (100 mg), sulfanilamide (53 mg), p-toluenesulfonic acid (53 mg) and sec-butanol (5 ml) were added into a round bottom flask. The reaction system was stirred at 120°C overnight. After the completion of the reaction, the reaction mixture was filtered, and washed with methanol and diethyl ether to obtain compound 1. LC/MS: M+H 461.1.

### 2. Preparation of other MST1/2 inhibitor compounds of the invention

Other MST1/2 inhibitor compounds of the invention were synthesized via the method similar to that of Compound 1, and their structures and mass spectrum data are shown in the following table.

| No. | Compound | MS(ESI) m/z(M+1)+ | No. | Compound | MS(ESI) m/z(M+1)+ |
|---|---|---|---|---|---|
| 1 | 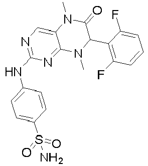 | 461.1 | 2 | 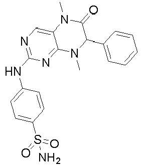 | 425.14 |
| 3 | 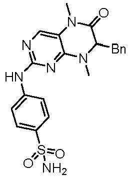 | 439.15 | 4 | 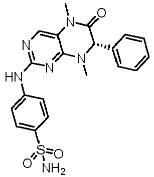 | 425.14 |
| 5 | 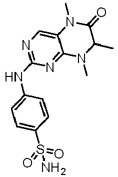 | 363.12 | 6 | 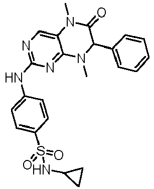 | 465.17 |
| 7 | 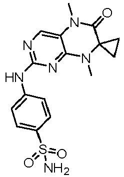 | 375.12 | 8 | 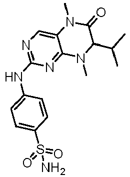 | 391.16 |
| 9 | 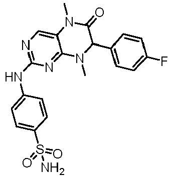 | 443.13 | 10 | 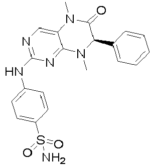 | 425.14 |
| 11 | 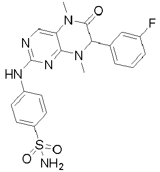 | 443.13 | 12 | 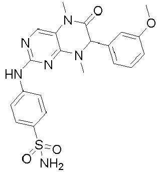 | 455.15 |
| 13 | 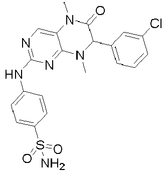 | 459.10 | 14 | 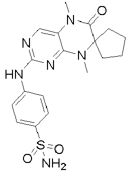 | 403.15 |
| 15 | 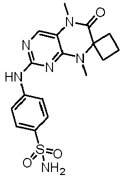 | 389.14 | 16 | 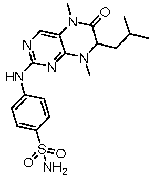 | 405.17 |
| 17 | 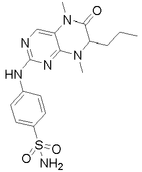 | 391.15 | 18 | 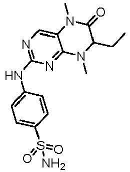 | 377.14 |
| 19 | 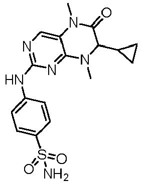 | 389.14 | 20 | 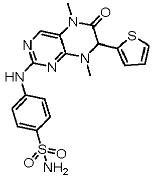 | 431.09 |
| 21 | 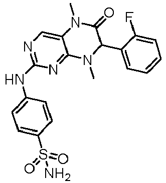 | 443.13 | 22 | 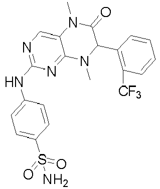 | 493.12 |
| 23 | 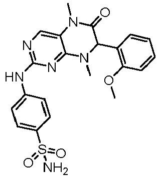 | 455.15 | 24 | 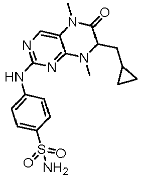 | 403.15 |
| 25 | 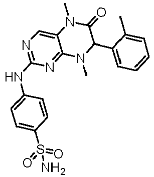 | 439.15 | 26 | 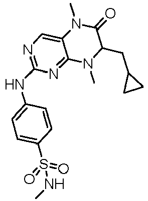 | 417.17 |
| 27 | 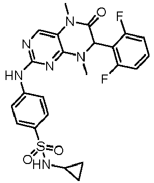 | 501.15 | 28 | 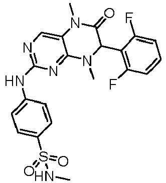 | 475.13 |
| 29 | 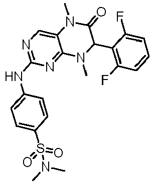 | 489.15 | 30 | 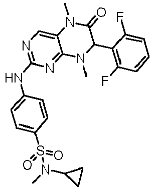 | 515.16 |
| 31 | 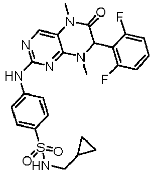 | 515.16 | 32 | 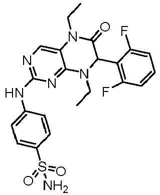 | 489.15 |
| 33 | 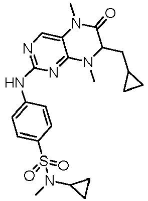 | 457.20 | 34 | 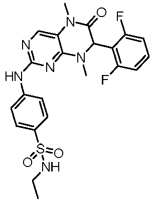 | 489.15 |
| 35 | 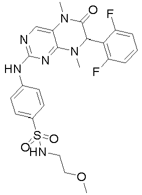 | 519.16 | 36 | 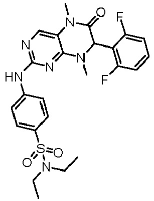 | 517.18 |
| 37 | 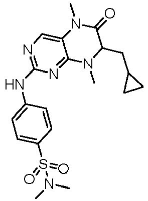 | 431.18 | 38 | 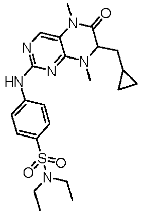 | 459.21 |
| 39 | 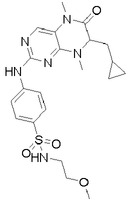 | 461.19 | 40 | 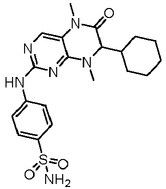 | 431.19 |
| 41 | 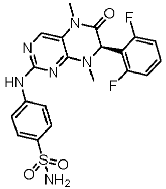 | 461.12 | 42 | 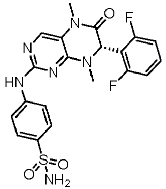 | 461.12 |
| 43 | 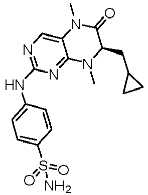 | 403.15 | 44 | 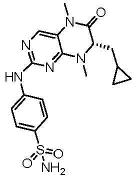 | 403.15 |
| 45 | 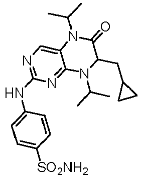 | 459.21 | 46 | 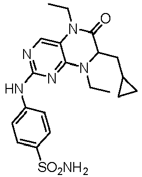 | 431.18 |
| 47 | 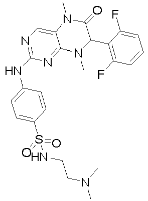 | 532.19 | 48 | 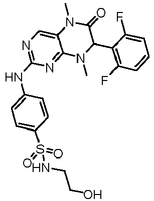 | 505.14 |
| 49 | 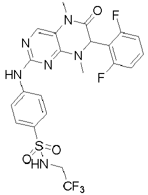 | 543.12 | 50 | 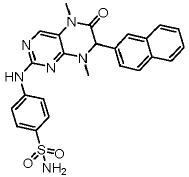 | 475.16 |
| 51 | 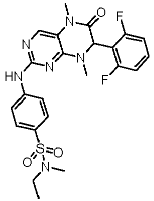 | 503.17 | 52 | 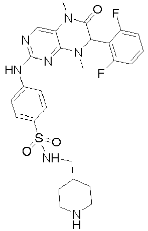 | 558.21 |
| 53 | 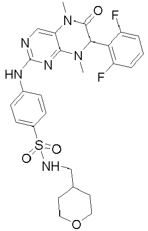 | 559.19 | 54 | 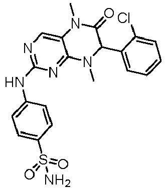 | 459.10 |
| 55 | 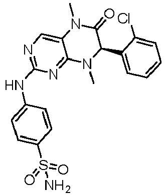 | 459.10 | 56 | 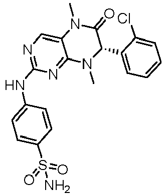 | 459.10 |
| 57 | 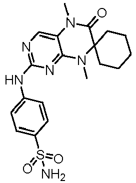 | 417.17 | 58 | 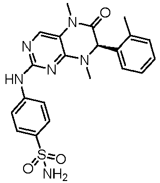 | 439.16 |
| 59 | 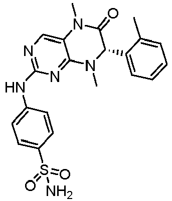 | 439.16 | | | |

### [Example 1]

### Isolation of Human Primary Lung Cancer Epithelial Cells

Lung cancer tissue samples were derived from the cancer tissue samples by surgical resection from lung cancer patients who have given informed consent. One of the samples (No. B4) are described as below.

The aforementioned tissue samples were collected within half an hour after a surgical resection. More specifically, in a sterile environment, tissue samples from non-necrotic sites were cut with a volume of 0.5 cm³ or more, and were placed in 4 ml of pre-cooled tissue transport fluid (specific formulation shown in Table 1). The transport fluid was placed in a 5 ml plastic sterile cryopreservation tube with a lid (purchased from Guangzhou Jet Bio-Filtration Co., Ltd.) and cold chain (0-10°C) transported to the laboratory.

**Table 1. Formulation of tissue transport fluid**

| Components of tissue transport fluid | Supplier | Final concentration |
|---|---|---|
| DMEM/F12 | Corning | 97.8 vol.% |
| Primocin | Invivogen | 0.2 vol.% (concentration of commercial product: 50 mg/ml) |
| Compound 1 | Self prepared | 3 µM |

**Table 2. Formulation of tissue digestive solution**

| Components of tissue digestive solution | Supplier | Final concentration |
|---|---|---|
| HBSS | Gibco | 50 vol.% |
| RPMI-1640 | Corning | 50 vol.% |
| collagenase II | Sigma | 2 mg/ml |
| collagenase IV | Sigma | 2 mg/ml |
| deoxyribonucleic acid I | Sigma | 50 U/ml |
| hyaluronidase | Sigma | 0.5 mg/ml |
| calcium chloride | Sangon Biotech | 0.33 mg/ml |
| bovine serum albumin | Sangon Biotech | 10 mg/ml |

In the biological safety cabinet, the tissue sample (No. B4) was transferred to a 100 mm cell-culture dish (purchased from NEST). The tissue sample was rinsed with the tissue transport fluid. The residual blood on the surface of the tissue sample was washed away. Excess tissues such as fat on the surface of the tissue sample were removed. The rinsed tissue sample was transferred to another new 100 mm culture dish; 2 ml of transport fluid was added, and a sterile scalpel blade and a forceps were used to divide the tissue sample into tissue fragments less than 3 mm³ in volume.

The tissue sample fragments were transferred to a 15 ml centrifuge tube, and centrifuged at 1500 rpm for 4 minutes in a tabletop centrifuge (Sigma, 3-18K); after discarding the supernatant, the tissue transport fluid and the tissue digestive solution were added in a ratio of 1:1 (the dosage is about 5 ml of tissue digestive solution per 10 mg of tissue; specific formulation was shown in Table 2); then the sample was numbered and sealed with sealing film, and was then digested in a constant-temperature shaker (Zhichu Instrument ZQLY-180N) at 37°C, 300 revolutions; whether the digestion was completed was determined via observation every 1 hour.

After digestion, undigested tissue blocks were filtered out by a 70 µm filter screen; the tissue blocks on the filter screen were rinsed with the tissue transport fluid; the residual cells were rinsed into a centrifuge tube and centrifuged at 1500 rpm for 4 minutes.

After discarding the supernatant, the remaining cell pellet was observed to determine whether blood cells were remained; if there were blood cells, 3 ml blood cell lysate (purchased from Sigma) was added, which was then mixed well, lysed at 4°C for 15 minutes, with shaking and mixing well once every 5 minutes; after lysis, the resultant was take out and centrifuged at 1500 rpm for 4 minutes. The supernatant was discarded to provide digested and isolated primary lung cancer cells, which were added with basic medium (BM) for resuspension. The basic medium was prepared by adding 0.2 vol.% of Primocin (purchased from Invivogen, with a concentration of 50 mg/ml) to the commercial DMEM/F-12 medium to provide a final concentration of 100 µg/ml. The total number of cells was 1,020,000, which was obtained by counting with a flow imaging counter (JIMBIO FIL, Jiangsu Jimbio Technology Co., Ltd.).

### [Example 2]

### Optimization of Culture Medium for Primary Lung Cancer Epithelial Cells

### (1) Effects of different factors

Extracellular matrix gel (Matrigel^{®}) (manufactured by Corning) was diluted with serum-free DMEM/F12 medium at a ratio of 1:100 to prepare an extracellular matrix diluent. The extracellular matrix diluent was added to a 48-well culture plate with 500 µl/well to completely cover the bottom of the wells of the culture plate. The culture plate was let stand for 1 hour in a 37°C incubator. After 1 hour, the extracellular matrix diluent was removed to provide a Matrigel-coated plate.

Preparation of basic medium (abbreviated as BM): BM was prepared by adding 0.2 vol.% of Primocin (purchased from Invivogen, with a concentration of 50 mg/ml) to the commercial DMEM/F-12 medium to provide a final concentration of 100 µg/ml.

Next, different kinds and concentrations of additive factors (Table 3) were added to the basic medium (BM), so as to prepare culture mediums for lung cancer epithelial cells containing different additive components.

**Table 3. Preparation of culture mediums containing different components (final concentrations are shown)**

| Culture medium | Sources of additive factors | Composition |
|---|---|---|
| Basic medium / BM | | DMEM/F12+100 µg/mL Primocin |
| BM + gastrin | Sino Biological Inc. | BM+80, 40, 20, 10, 5, 2.5 ng/ml gastrin |
| BM + epidermal growth factor (EGF) | Sino Biological Inc. | BM+80, 40, 20, 10, 5, 2.5 ng/ml EGF |
| BM + transferrin | Sino Biological Inc. | BM+80, 40, 20, 10, 5, 2.5 ng/ml transferrin |
| BM + fibroblast growth factor (FGF) | Sino Biological Inc. | BM+80, 40, 20, 10, 5, 2.5 ng/ml FGF |
| BM + N2 additive | Gibco | BM+1/25, 1/50, 1/100, 1/200, 1/400, 1/800 diluting ratio of N2 |
| BM + B27 additive | Gibco | BM+1/25, 1/50, 1/100, 1/200, 1/400, 1/800 diluting ratio of B27 |
| BM + Y27632 | MCE | BM+40, 20, 10, 5, 2.5, 1.25 µM Y27632 |
| BM + Compound 1 | Preparation Example | BM+40, 20, 10, 5, 2.5, 1.25 µM Compound 1 |
| BM + A83-01 | MCE | BM+4000, 2000, 1000, 500, 250, 125 nM A83-01 |

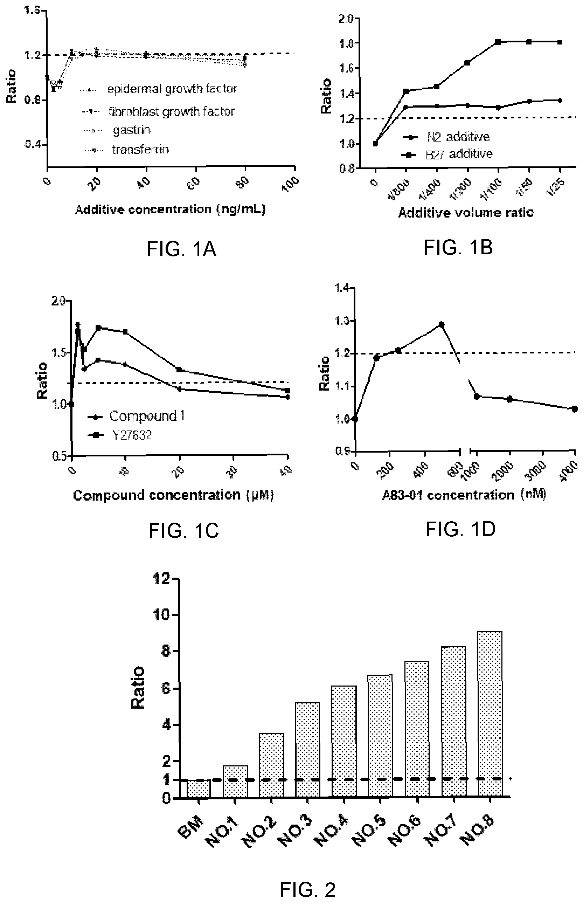

The culture mediums with different components were added at a volume of 500 µl/well to 48-well plates which were coated with extracellular matrix gel (Matrigel). Lung cancer cells (No. B18) isolated from lung cancer tissues according to the same method as described in Example 1 were inoculated at a cell density of 2×10⁴ cells/cm² in the above-mentioned 48-well culture plates which were coated with Matrigel. After surface disinfection, the plates were placed in a 37°C, 5% CO₂ incubator (purchased from Thermo Fisher), and the same number of freshly isolated lung cancer tumor cells (No. B18) were cultured under different medium formulations. The culture mediums were replaced every 4 days after the start of culture. After 12 days of culture, cell counts were performed. The basic medium (BM) without addition of any additive factor was used as a control. The results were shown in Figures 1A-1D. The ordinate in the figures represents the ratio of the number of cells obtained after culture in different mediums to the number of cells obtained after culture in basic medium BM. As shown in the figures, adding different concentrations of different factors according to Table 3 to BM all resulted in the proliferation of cells but with varying degrees. Specifically, B27 additive, N2 additive, fibroblast growth factor, gastrin, epidermal growth factor, transferrin, Compound 1, Y27632 and A83-01 provided certain promoting effects on cell proliferation in specific concentration ranges.

### (3) Effects of different increasing factors in the culture mediums on the proliferation of primary lung cancer cells obtained by the method of the invention

Extracellular matrix gel (Matrigel^{®}) was diluted with serum-free DMEM/F12 medium at a ratio of 1:100 to prepare an extracellular matrix diluent. The extracellular matrix diluent was added to a 48-well culture plate with 500 µl/well to completely cover the bottom of the wells of the culture plate. The culture plate was let stand for 1 hour in a 37°C incubator. After 1 hour, the extracellular matrix diluent was removed to provide a Matrigel-coated plate.

Different additive factors (Table 4) were sequentially added to the basic medium BM, respectively, so as to prepare culture mediums for lung cancer epithelial cells containing different additive components.

**Table 4. Preparation of culture mediums containing different components (final concentrations are shown)**

| Medium NO. | Component |
|---|---|
| NO.1 | BM + 10 µM Y27632 |
| NO.2 | NO.1 + 5 µM Compound 1 |
| NO.3 | NO.2 + 1:50 B27 Additive |
| NO.4 | NO.3 + 40 ng/ml fibroblast growth factor |
| NO.5 | NO.4 + 40 ng/ml epidermal growth factor |
| NO.6 | NO.5 + 40 ng/ml transferrin |
| NO.7 | NO.6 + 500 nM A83-01 |
| NO.8 | NO.7 + 20 ng/ml gastrin |

The culture mediums with different components were added at a volume of 500 µl/well to 48-well plates which were coated with extracellular matrix gel (Matrigel), and simultaneously, the BM medium was used as a control. Lung cancer cells (No. B22) isolated from lung cancer tissues according to the method described in Example 1 were inoculated at a cell density of 2×10⁴cells/cm² in the 48-well culture plates which were coated with Matrigel. After surface disinfection, the plates were placed in a 37°C, 5% CO₂ incubator (purchased from Thermo Fisher), and the same number of freshly isolated lung cancer tumor cells (No. B22) were cultured under different medium formulations. After 10 days of culture, cell counts were performed. The results were shown in Figure 2. As shown in the figure, with the sequential addition of new additive factors, the cell proliferation effect of the culture medium formulation was continuously improved, and it was finally determined that No. 8 culture medium formulation was the most preferable culture medium in this application for culturing and expanding primary lung cancer cells.

### (4) Effects of different concentrations of the additive factors on the proliferation of primary lung cancer cells obtained in the invention

Extracellular matrix gel (Matrigel^{®}) was diluted with serum-free DMEM/F12 medium at a ratio of 1:100 to prepare an extracellular matrix diluent. The extracellular matrix diluent was added to a 48-well culture plate with 200 µl/well to completely cover the bottom of the wells of the culture plate. The culture plate was let stand for 1 hour in a 37°C incubator. After 1 hour, the extracellular matrix diluent was removed to provide a Matrigel-coated plate.

No. 8 culture medium for primary lung cancer epithelial cells was prepared.

Lung cancer epithelial cells derived from cancer tissues were isolated from the cancer tissue of a lung cancer patient (Sample No. B26) using the same method as in Example 1. Next, lung cancer epithelial cells derived from cancer tissues were counted with a flow imaging counter (JIMBIO FIL, Jiangsu Jimbio Technology Co., Ltd.) to get the total number of cells. Then, the cells were inoculated at a density of 4×10⁴ cells/cm² in a 48-well plate which was coated with Matrigel^{®}. 2 ml of the prepared No. 8 culture medium for primary lung cancer epithelial cells was added to the 48-well plate, which was then placed in a 37°C, 5% CO₂ incubator (purchased from Thermo Fisher) for culture. When the cells in the culture plate grew to cover about 80% of the bottom area, the supernatant of medium in the 48-well plate was discarded and 500 µl of 0.05% trypsin (purchased from Gibco) was added for cell digestion, which was then incubated at 37°C for 10 minutes, until the cells were completely digested as observed under a microscope (EVOS M500, Invitrogen); then the digestion was terminated by using 1 ml of DMEM/F12 culture solution containing 5% (v/v) fetal bovine serum (purchased from ExCell Bio), 100 U/ml penicillin (purchased from Corning), and 100 µg/ml streptomycin (purchased from Corning); the resultant was collected into a 15 ml centrifuge tube and centrifuged at 1500 rpm for 4 minutes, and then the supernatant was discarded. The centrifuged cell pellet was resuspended in the basic medium BM and the cells were counted with a flow imaging counter (JIMBIO FIL, Jiangsu Jimbio Technology Co., Ltd.) to get the total number of cells. The obtained cells were used in the following cultivation experiments.

Next, the following 8 formulations of culture medium were prepared for experiments:
Formulation 1: No. 8 culture medium composition without B27 additive;
Formulation 2: No. 8 culture medium composition without fibroblast growth factor;
Formulation 3: No. 8 culture medium composition without transferrin;
Formulation 4: No. 8 culture medium composition without epidermal growth factor;
Formulation 5: No. 8 culture medium composition without Y27632;
Formulation 6: No. 8 culture medium composition without Compound 1;
Formulation 7: No. 8 culture medium composition without A83-01;
Formulation 8: No. 8 culture medium composition without gastrin.

The digested cell suspension was diluted with the above Formulations 1-8 respectively, and then inoculated into a 48-well plate at a volume of 250 µl with 10,000 cells per well.

When using the medium of Formulation 1, to a 48-well plate inoculated with primary cells was added the prepared B27 additive, with 250 µl per well, the final concentrations of B27 additive were 1:800, 1:400, 1:200, 1:100, 1:50, 1:25, respectively; a well of Blank Control (BC) was set using the medium of Formulation 1.

When using the medium of Formulation 2, to a 48-well plate inoculated with primary cells was added the prepared fibroblast growth factor, with 250 µl per well, the final concentrations of fibroblast growth factor were 80 ng/ml, 40 ng/ml, 20 ng/ml, 10 ng/ml, 5 ng/ml, 2.5 ng/ml, respectively; a well of Blank Control (BC) was set using the medium of Formulation 2.

When using the medium of Formulation 3, to a 48-well plate inoculated with primary cells was added the prepared transferrin, with 250 µl per well, the final concentrations of transferrin were 80 ng/ml, 40 ng/ml, 20 ng/ml, 10 ng/ml, 5 ng/ml, 2.5 ng/ml, respectively; a well of Blank Control (BC) was set using the medium of Formulation 3.

When using the medium of Formulation 4, to a 48-well plate inoculated with primary cells was added the prepared epidermal growth factor, with 250 µl per well, the final concentrations of epidermal growth factor were 80 ng/ml, 40 ng/ml, 20 ng/ml, 10 ng/ml, 5 ng/ml, 2.5 ng/ml, respectively; a well of Blank Control (BC) was set using the medium of Formulation 4.

When using the medium of Formulation 5, to a 48-well plate inoculated with primary cells was added the prepared Y27632, with 250 µl per well, the final concentrations of Y27632 were 20 µM, 18 µM, 15 µM, 12.5 µM, 10 µM, 5 µM, 2.5 µM, respectively; a well of Blank Control (BC) was set using the medium of Formulation 5.

When using the medium of Formulation 6, to a 48-well plate inoculated with primary cells was added the prepared Compound 1, with 250 µl per well, the final concentrations of Compound 1 were 20 µM, 15 µM, 10 µM, 7.5 µM, 5 µM, 2.5 µM, 1.25 µM, respectively; a well of Blank Control (BC) was set using the medium of Formulation 6.

When using the medium of Formulation 7, to a 48-well plate inoculated with primary cells was added the prepared A83-01, with 250 µl per well, the final concentrations of A83-01 were 2000 nM, 1000 nM, 800 nM, 500 nM, 250 nM, 125 nM, 62.5 nM, respectively; a well of Blank Control (BC) was set using the medium of Formulation 7.

When using the medium of Formulation 8, to a 48-well plate inoculated with primary cells was added the prepared gastrin, with 250 µl per well, the final concentrations of gastrin were 80 ng/ml, 40 ng/ml, 20 ng/ml, 10 ng/ml, 5 ng/ml, 2.5 ng/ml, respectively; a well of Blank Control (BC) was set using the medium of Formulation 8.

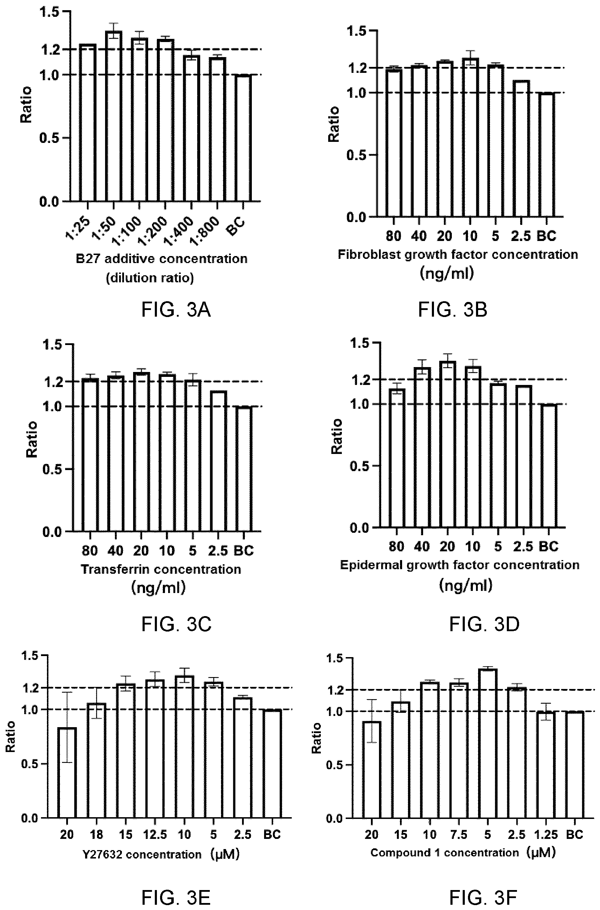

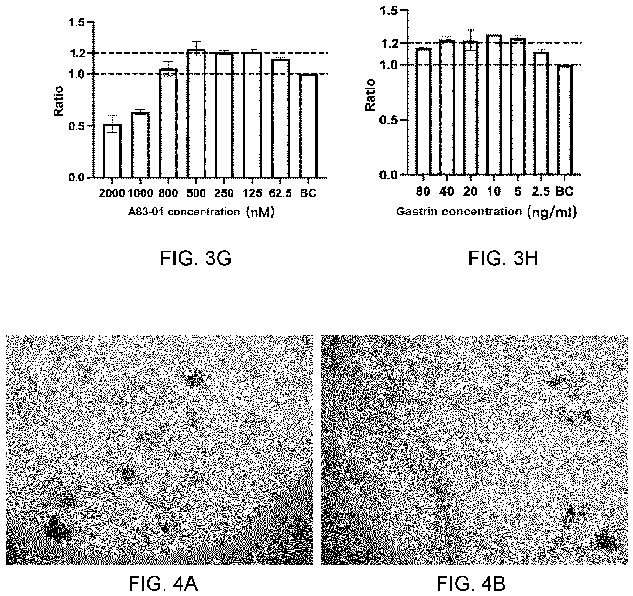

After the cells were expanded to about 85% of the 48 wells, the cells were digested and counted, the ratios were calculated by referring to the cell numbers in the well of Blank Control (BC), and the results were shown in Figures 3A-3H. In each of Figures 3A-3H, the ratio represents a ratio of the number of cells of the first passage cultured by using each culture medium to the number of cells of the first passage cultured by the corresponding well of Blank Control. If the ratio is greater than 1, it indicates that the proliferation promoting effect of the prepared medium containing different concentrations of factors or small molecular compounds is preferable over that of the medium in the well of Blank Control; if the ratio is less than 1, it indicates that the proliferation promoting effect of the prepared medium containing different concentrations of factors or small molecular compounds is poorer than that of the medium in the well of Blank Control.

According to the results of Figures 3A -3H, the volume concentration of B27 additive in the culture medium is preferably 1:25-1:800, more preferably 1:25-1:200, and most preferably 1:50; the amount of fibroblast growth factor is preferably 2.5 ng/ml-80 ng/ml, more preferably 5 ng/ml-40 ng/ml, and most preferably 10 ng/ml; the amount of transferrin is preferably 2.5 ng/ml-80 ng/ml, more preferably 5 ng/ml-80 ng/ml, and most preferably 20 ng/ml; the amount of epidermal growth factor is preferably 2.5 ng/ml-80 ng/ml, more preferably 10 ng/ml-40 ng/ml, and most preferably 20 ng/ml; the amount of Y27632 is preferably 2.5 µM-18 µM, more preferably 5 µM-15 µM, and most preferably 10 µM; the amount of MST1/2 kinase inhibitor Compound 1 is preferably 2.5 µM-15 µM, more preferably 2.5 µM-10 µM, and most preferably 5 µM; the amount of A83-01 is preferably 62.5 nM-800 nM, more preferably 125 nM-500 nM, and most preferably 500 nM; the amount of gastrin is preferably 2.5 ng/ml-80 ng/ml, more preferably 5 ng/ml-40 ng/ml, and most preferably 10 ng/ml.

The optimal concentration combination of various additive factors was used as the most preferred culture medium formulation for culturing and expanding primary lung cancer cells in the invention (hereinafter referred to as FLM): basic medium (BM) + 10 ng/ml fibroblast growth factor (FGF) + 20 ng/ml epidermal growth factor (EGF) + 20 ng/ml transferrin + 1:50 volume ratio of B27 additive + 5 µM Compound 1 + 10 µM Y27632 + 500 nM A83-01 + 10 ng/ml gastrin.

### [Example 3]

### Culture of Primary Lung Cancer Cells Derived From Lung Cancer Tissues

Lung cancer epithelial cells derived from cancer tissues were isolated from cancer tissues of lung cancer patients (Sample No. B21) using the same method as in Example 1. Next, cancer tissue-derived lung cancer epithelial cells were counted with a flow imaging counter (JIMBIO FIL, Jiangsu Jimbio Technology Co., Ltd.) to get the total number of cells. Then, the cells were inoculated in a 12-well plate which was coated with Matrigel^{®} (purchased from BD Biosciences) at a density of 4×10⁴ cells/cm². 2 ml of the prepared culture medium FLM for primary lung cancer epithelial cells was added to the 12-well plate, which was then placed in a 37°C, 5% CO₂ incubator (purchased from Thermo Fisher) for culture.

Figure 4A is a microscopy image (photographed by a 40x inverted phase contrast microscope) of the cells which were cultured to day 4 after inoculation at a density of 4×10⁴ cells/cm² into the 12-well plate which was coated with Matrigel. Microscopic observation shows that the cultured primary lung cancer cells derived from cancer tissues had high purity, without fibroblasts. Figure 4B is a microscopy image (photographed by a 40x inverted phase contrast microscope) of the cells which were cultured to day 12 after inoculation. It can be seen from Figures 4A and 4B that the formation of obvious clone can be seen under microscope when the primary lung cancer cells isolated were cultured *in vitro* for 4 days, and the number of cells was significantly expanded after 12 days of expansion, suggesting that the culture medium of the invention is an efficient culture medium for expanding lung cancer epithelial cells *in vitro.*

### [Example 4]

Effects of Different Culture Mediums on Promoting Proliferation of Lung Cancer Tissue-Derived Primary Lung Cancer Cells

### (1) Comparison of the influences of different culture mediums on clone formation of primary cells of the first generation and comparison of proliferation effects thereof

Culture medium FLM for primary lung cancer epithelial cell was prepared in the same method of Example 2, and basic medium BM was prepared as control. As another control, a culture medium FM used in the cell conditional reprogramming technique was prepared additionally. For the preparation steps, see Liu et al., Nat Protoc., 12(2): 439-451, 2017. The formulation of the culture medium is shown in Table 5. Moreover, as an additional control, a commercial medium EpiMCult^{™} Plus Medium (hereinafter also referred to as "EpiM medium") was purchased from stem cell, and the formulation of the culture medium is shown in Table 6.

**Table 5. Composition of culture medium FM used in the cell conditional reprogramming technique**

| Medium composition | Supplier | Final concentration |
|---|---|---|
| DMEM medium | Corning | 65 vol.% |
| Fetal bovine serum | Gibico | 10 vol.% |
| Ham's F12 Nutrient Solution | Gibico | 25 vol.% |
| Hydrocortisone | Sigma-Aldrich | 25 ng/ml |
| Epidermal growth factor | R&D | 0.125 ng/ml |
| Insulin | Sigma-Aldrich | 5 µg/ml |
| Amphotericin B | Sigma-Aldrich | 250 ng/ml |
| Gentamicin | Gibico | 10 µg/ml |
| Cholera Toxin | Sigma-Aldrich | 0.1 nM |
| Y27632 | Enzo | 10 µM |

**Table 6. Composition of commercial medium EpiMCult^{™} Plus Medium (EpiM)**

| Medium composition | Supplier | Final concentration |
|---|---|---|
| EpiMCult^{™} Plus Basal Medium | stem cell | 98 vol.% |
| EpiMCult^{™} Plus Supplement | stem cell | 2 vol.% |
| hydrocortisone | stem cell | 480 ng/ml |

By using the same method of Example 1, the primary lung cancer cells (No. B8) derived from lung cancer tissues were obtained. Next, the cells were cultured at the same density (4×10⁴ cells/cm²) under the following 5 culture conditions:
A. The primary lung cancer cells were inoculated into a 24-well plate which was coated with Matrigel^{®} at a density of 4×10⁴ cells/cm², cultured using 2 ml of the culture medium FLM for primary lung cancer epithelial cells of the invention;
B. The primary lung cancer cells were inoculated into a 24-well plate which was pre-laid with γ-ray irradiated Mouse fibroblast cell line J2 cells (purchased from Kerafast) at a density of 4×10⁴ cells/cm², cultured in the 24-well plate using the cell conditional reprogramming medium FM (the detailed steps, see Liu et al., Am J Pathol, 183(6): 1862-1870, 2013);
C. The primary lung cancer cells were inoculated into a 24-well plate which was coated with Matrigel^{®} at a density of 4×10⁴ cells/cm², and cultured in the 24-well plate using 2 ml of the commercial culture medium EpiM;
D. The primary lung cancer cells were inoculated into a 24-well plate which was coated with Matrigel^{®} at a density of 4×10⁴ cells/cm², and cultured in the 24-well plate using 2 ml of the basic medium BM.

In the above four cultures, the cells cultured under the four culture conditions wherein the mediums were renewed every 4 days. At the same time, the cell clone formation and the cell proliferation status under the cultivation of each medium in the 24-well plate was observed, and the cell growth status was recorded by taking pictures with a microscope (EVOS M500, Invitrogen).

Regarding the primary lung cancer cells (No. B8) cultured with the technique of the invention, when the cells in the culture plate grew to cover about 80% of the bottom area, the medium supernatant in the 24-well plate was discarded and 500 µl of 0.05% trypsin (purchased from GIBCO) was added for cell digestion, which was then incubated at 37°C for 10 minutes until the cells were completely digested as observed under the microscope (EVOS M500, Invitrogen); then the digestion was terminated by using 1 ml of DMEM/F12 culture solution containing 5% (v/v) fetal bovine serum, 100 U/ml penicillin, and 100 µg/ml streptomycin; the resultant was collected into a 15 ml centrifuge tube and centrifuged at 1500 rpm for 4 minutes, and then the supernatant was discarded. The centrifuged cell pellet was resuspended in the culture medium of the invention and the cells were counted with a flow imaging counter (JIMBIO FIL, Jiangsu Jimbio Technology Co., Ltd.) to obtain the total number of cells, which was 464,000. The cells cultured under the other three culture conditions were digested and counted in the same operation process as above. The total number of cells cultured by using the mediums FM, EpiM and BM were 350,000, 110,000 and 68,000, respectively.

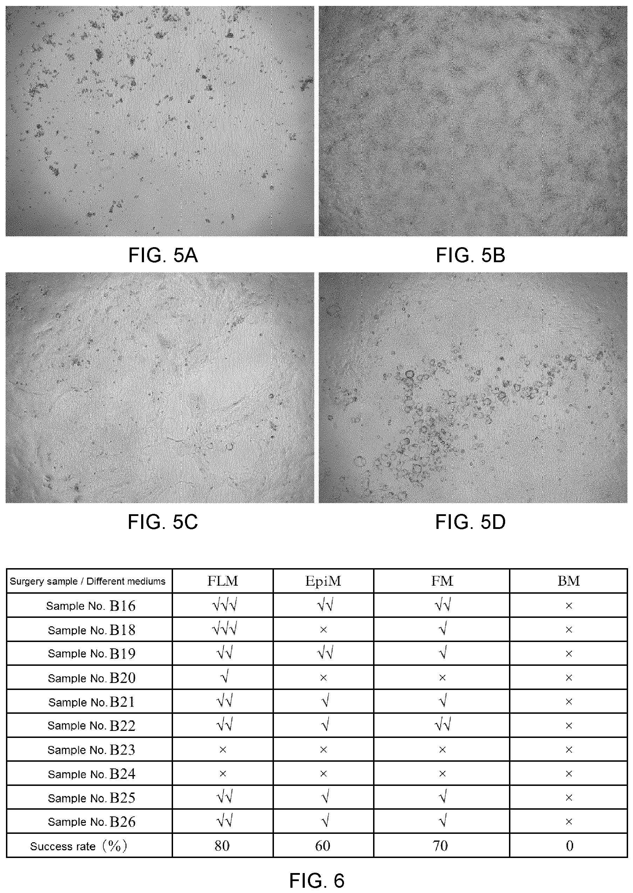

The cell photos in Figures 5A-5D are microscopic photos (under a 40x inverted phase contrast microscope) of the sample numbered B8 cultured under four different culture conditions until day 12: Figure 5A is the microscopic photo of B8 cultured to day 12 using basic medium BM; Figure 5B is the microscopic photo of B8 cultured to day 12 using the culture medium FLM of the invention; Figure 5C is the microscopic photo of B8 cultured to day 12 using the commercial culture medium EpiM; Figure 5D is the microscopic photo of B8 cultured to day 12 using the conditioned reprogramming medium FM. As can be seen from the figures, sample B8 cannot form cell clones when cultured with basic medium BM (Figure 5A) for 12 days; only a small number of cell clones are formed when cultured with EpiM (Figure 5C) for 12 days, and the cells are in poor condition; the cells show certain expansion when cultured with the conditional reprogramming medium FM (Figure 5D) for 12 days, but the cell density and cell number are not comparable to those in the medium FLM of the invention (Figure 5B).

Figure 6 is a comparative diagram of cell proliferation effects obtained by culturing the primary lung cancer cells isolated from ten lung cancer patient samples according to Example 1 for 14 days under the conditions of four different culture mediums, where V represents a moderate clone formation ability and proliferation promoting effect, √√ represents a significant clone formation ability and proliferation promoting effect, √√√ represents an extremely significant clone formation ability and proliferation promoting effect, and × represents no clone formation. It can be confirmed from Figure 6 that the culture medium FLM of the invention has significant superiority over the other three culture conditions in terms of clone formation ability, cell proliferation promoting effect and successful rate of culture in cultivation of the lung cancer tissue-derived primary cells.

### (2) Continuous cultivation and growth curve of primary lung cancer cells in different culture mediums

The culture medium FLM for primary lung cancer epithelial cells and the culture mediums BM, FM, and EpiM as controls were obtained by using the same method as in (1) of this Example.

The lung cancer tissue-devired primary lung cancer cells (No. B16) were cultured under four culture conditions by using the same method as in (1) of this Example, and then digested, passaged and counted.

When the passaged cells grew in the culture plate to cover about 80% of the bottom area of the plate again, the cultured cells were digested, collected and counted according to the above operating method. The cells were inoculated at a density of 4×10⁴ cells/well again and cultured continuously.

The following is the formula for calculating the cell population doubling number of primary lung cancer epithelial cells under different culture conditions: Population Doubling (PD) = 3.32 * log₁₀ (total number of digested cells / the number of cells at initial inoculation), see Chapman et al., Stem Cell Research & Therapy 2014, 5: 60.

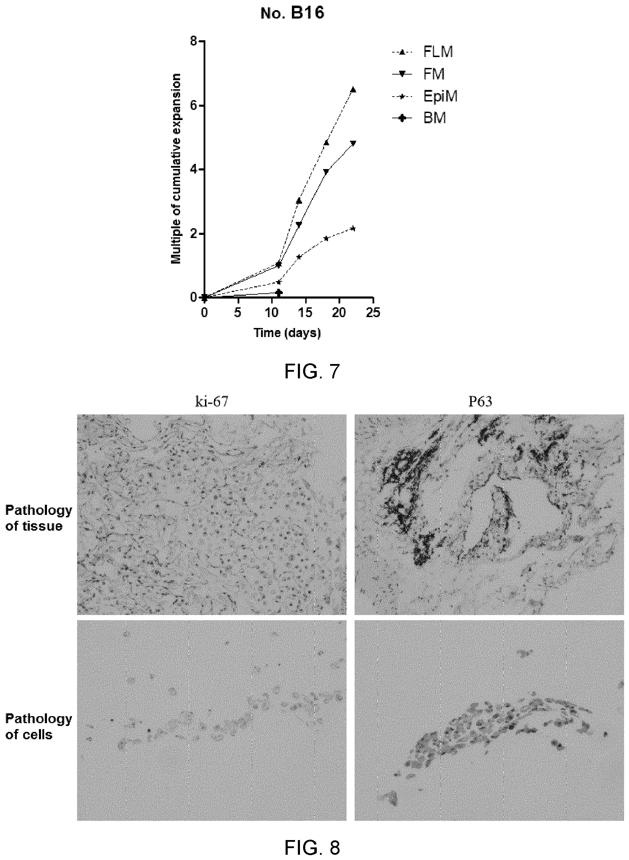

Figure 7 shows the growth curves of B16 cells under four different culture conditions drawn by Graphpad Prism software. The abscissa represents the days of cell culture, and the ordinate represents the multiple of cumulative cell expansion, i.e., the multiple of cell expansion in the culture period. The larger the value, the more multiples the cell expands in certain period, that is, the more cells are expanded. The slope represents the rate of cell expansion. It can be confirmed from the figure that the proliferation rates of lung cancer epithelial cells cultured with the culture medium FLM of the invention was superior to the other four culture conditions, and it can also be confirmed that the culture medium of the invention can continuously culture the primary lung cancer epithelial cells, and the rate remains unchanged for expanding of more than 20 days.

### [Example 5]

Immunohistochemical Identification of Primary Lung Cancer Tissues and Lung Cancer Cells after Passage Culture

Cancer tissue (Sample No. B16) about the size of a mung bean was taken from a surgical resection sample of a lung cancer patient, and immersed in 1 ml of 4% paraformaldehyde. Lung cancer epithelial cells (Sample No. B16) were obtained from the remaining cancer tissue in the same method of Example 1. Sample B16 was continuously cultured to the fourth passage using the culture medium FLM of the invention according to the method of Example 3.

Immunohistochemistry assay was used to detect the expression of important lung cancer-related biomarkers in the original tissue of Sample B16 and the primary cells obtained by continuous culture to the fourth passage. The tissue was fixed with 4% paraformaldehyde, embedded in paraffin, and cut into tissue sections of 4 µm thickness with a microtome. Routine immunohistochemical detection was then performed (for detailed steps, see Li et al., Nature Communication, (2018) 9: 2983). The primary antibodies used were P63 antibody (purchased from CST) and Ki67 antibody (purchased from R&D).

Figure 8 confirms that the expression of lung cancer-related biomarkers on the cells that were cultured from the lung cancer cells (Sample No. B16) with the culture medium of the invention to the 4th passage, was substantially consistent with the expression of markers on the original tissue section from which the cells were derived. This suggests that the cells cultured with the culture medium of the invention maintain the original pathological characteristics of the cancer tissues of the lung cancer patients.

### [Example 6]

Effects of Removing a Single Factor from the Culture Medium on the Continuous Expansion and Culture of Primary Lung Cancer Cells

The culture medium FLM for primary lung cancer epithelial cells was prepared using the same method as Example 2. As a control, the same method as Example 2 was used to prepare basic medium BM. In addition, other 8 different culture mediums were prepared according to Table 7.

**Table 7 Culture mediums with different compositions (final concentrations are shown)**

| Culture Medium | Composition |
|---|---|
| FLM without Compound 1 | DMEM/F12 + 100 µg/mL Primocin + 1:50 B27 additive + 10 ng/ml fibroblast growth factor + 20 ng/ml epidermal growth factor + 20 ng/ml transferrin + 10 µM Y27632 + 500 nM A83-01 + 10 ng/ml gastrin |
| FLM without Y27632 | DMEM/F12 + 100 µg/mL Primocin + 1:50 B27 additive + 10 ng/ml fibroblast growth factor + 20 ng/ml epidermal growth factor + 20 ng/ml transferrin + 5 µM Compound 1 + 500 nM A83-01 + 10 ng/ml gastrin |
| FLM without A83-01 | DMEM/F12 + 100 µg/mL Primocin + 1:50 B27 additive + 10 ng/ml fibroblast growth factor + 20 ng/ml epidermal growth factor + 20 ng/ml transferrin + 10 µM Y27632 + 5 µM Compound 1 + 10 ng/ml gastrin |
| FLM without fibroblast growth factor | DMEM/F12 + 100 µg/mL Primocin + 1:50 B27 additive + 5 µM Compound 1 + 20 ng/ml epidermal growth factor + 20 ng/ml transferrin + 10 µM Y27632 + 500 nM A83-01 + 10 ng/ml gastrin |
| FLM without transferrin | DMEM/F12 + 100 µg/mL Primocin + 1:50 B27 additive + 10 ng/ml fibroblast growth factor + 20 ng/ml epidermal growth factor + 5 µM Compound 1 + 10 µM Y27632 + 500 nM A83-01 + 10 ng/ml gastrin |
| FLM without epidermal growth factor | DMEM/F12 + 100 µg/mL Primocin + 1:50 B27 additive + 10 ng/ml fibroblast growth factor + 5 µM Compound 1 + 20 ng/ml transferrin + 10 µM Y27632 + 500 nM A83-01 + 10 ng/ml gastrin |
| FLM without gastrin | DMEM/F12 + 100 µg/mL Primocin + 1:50 B27 additive + 10 ng/ml fibroblast growth factor + 20 ng/ml epidermal growth factor + 20 ng/ml transferrin + 10 µM Y27632 + 500 nM A83-01 + 5 µM Compound 1 |
| FLM without B27 | DMEM/F12 + 100 µg/mL Primocin + 5 µM Compound 1 + 10 ng/ml fibroblast growth factor + 20 ng/ml epidermal growth factor + 20 ng/ml transferrin + 10 µM Y27632 + 500 nM A83-01 + 10 ng/ml gastrin |

One case of primary lung cancer cells (No. B18) derived from lung cancer tissues was obtained using the same method as in Example 1. The primary lung cancer cells were inoculated into a 48-well plate coated with Matrigel^{®} at a density of 4 × 10⁴ cells/cm², and cultured with 2 ml of the culture medium (FLM) for primary lung cancer epithelial cells of the invention, in a 37°C, 5% CO₂ incubator (purchased from Thermo Fisher).

When the cells in the culture plate grew to cover about 80% of the bottom area, the supernatant of medium in the 48-well plate was discarded, and 200 µl of 0.05% trypsin (purchased from Gibco) was added for cell digestion,which was then incubated at 37°C for 10 minutes, until the cells were completely digested as observed under a microscope (EVOS M500, Invitrogen); then the digestion was terminated by using 800 µl of DMEM/F12 culture solution containing 5% (v/v) fetal bovine serum, 100 U/ml penicillin, and 100 µg/ml streptomycin; the resultant was collected into a 15 ml centrifuge tube and centrifuged at 1500 rpm for 4 minutes, and then the supernatant was discarded. The centrifuged cell pellet was resuspended in the culture medium of the invention, and the cells were counted with a flow imaging counter (JIMBIO FIL, Jiangsu Jimbio Technology Co., Ltd.) to get the total number of cells. The cells were inoculated into another 48-well plate coated with extracellular matrix gel at a density of 2×10⁴ cells/cm² for further culture.

Cells cultured with the other 8 culture mediums and BM were digested, passaged and counted using the same methods as above, and cultured using different mediums.

When the passaged cells grew in the culture plate to cover about 80% of the bottom area of the plate again, the cultured cells were digested, collected and counted according to the above operating method. The cells were inoculated at a density of 2×10⁴ cells/cm² again and cultured continuously.

The following is the formula for calculating the cell population doubling number of primary lung cancer epithelial cells under different culture medium conditions:
Population Doubling (PD) = 3.32 * log₁₀ (total number of digested cells / the number of cells at initial inoculation), see Chapman et al., Stem Cell Research & Therapy 2014, 5: 60.

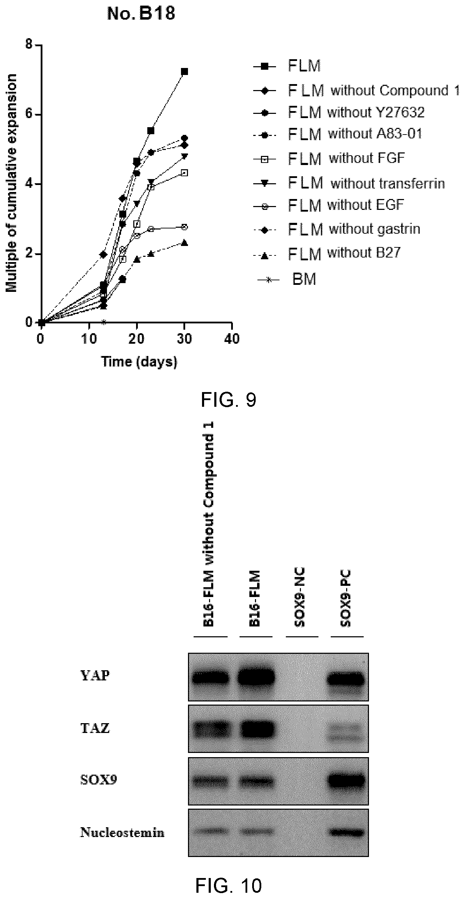

Figure 9 is a graph of cell growth curves drawn under ten different culture medium conditions using Graphpad Prism software. The abscissa represents the days of cell culture, and the ordinate represents the multiple of cumulative cell expansion, i.e., the multiple of cell expansion in the culture period. The larger the value, the more multiples the cell expands in certain period, that is, the more cells are expanded. The slope represents the rate of cell expansion.

It can be seen from the results in Figure 9 that after individually removing various small molecules and additive factors from the culture medium of the invention, the cell proliferation effect is weakened to a certain extent.

### [Example 7]

Freshly isolated primary lung cancer epithelial cell sample (No. B16) was obtained according to the steps described in Example 1. Then, the primary epithelial cells were inoculated onto a 6-well plate coated with Matrigel^{™}. 3mL of the culture medium FLM and 3mL of the culture medium FLM without Compound 1 were respectively added to the wells inoculated with the aforementioned epithelial cells. The plate was placed in a 37°C, 5% CO₂ incubator (purchased from Thermo Fisher) for culture. The culture mediums were changed every 4 days during the culture process. After 12 days of cultivation, cells of each group were collected and the expression of Compound 1 on Hippo pathway related proteins YAP and TAZ in the nucleus was detected using conventional immunoblotting method. Yes associated protein (YAP) and its homologous transcriptional co-activator PDZ-binding motif (TAZ) are key effectors in the Hippo pathway that control cell growth and organ size, and their dysregulation may lead to tumor development or hypertrophy. After activation, YAP/TAZ translocate to the nucleus and bind to TEAD transcription factors, promoting cell proliferation or regulated transcription process. Direct early genes represented by AP-1 complex are rapidly induced and control the late transcriptional process, playing a crucial role in tumor development and organ maintenance. The result suggests that Compound 1 can maintain the proliferation characteristics of lung cancer stem cells by inhibiting the MST1/2 mediated signaling pathway of lung tumor cells, and function to promote the sustained proliferation of lung cancer cells *in vitro.*

The testing result in Figure 10 shows that compared with the culture medium without Compound 1, the culture medium FLM with the addition of Compound 1 showed a significant increase in the expression of YAP and TAZ proteins in the nucleus. This indicates that Compound 1 can activate the Hippo pathway, making YAP/TAZ translocate to the nucleus and bind to TEAD transcription factors, and thus, promoting the sustained proliferation of cells.

### [Example 8]

### Xenograft Tumorigenesis Experiments of Cancer Tissue-Derived Primary Lung Cancer Cells in Mice

Lung cancer cells (No. B16) were isolated and obtained from the cancer tissues of one pathologically diagnosed lung cancer patient by using the same method as in Example 1. B16 was cultured using the culture medium FLM of the invention according to the method of Example 3, and when the number of lung cancer cells reached 1×10⁷, the lung cancer cells were digested and collected by using the same method of Example 4. The culture medium FLM for lung cancer cells of the invention and Matrigel^{®} were mixed at a ratio of 1:1, and 100 µl of the culture medium mixed with Matrigel was used to resuspend 5×10⁶ lung cancer cells, and the resultant was injected into the lung cancer fat pad and the axilla of the right forelimb of 6-week-old female highly immunodeficient mice (NCG) (purchased from Nanjing Model Animal Research Center), respectively. The volume and growth rate of tumors in mice generated from the lung cancer cells were observed and photographed every three days.

Tumor formation can be observed in both of the two tumor cell inoculation sites of the mice on day 15 after tumor cell inoculation. From day 15 to day 30, the tumor proliferation in mice was obvious. This indicates that the cancer tissue-derived lung cancer cells cultured by the culture method of the invention have tumorigenicity in mice.

### [Example 9]

### Drug Sensitivity Functional Test of Cancer Tissue-Derived Lung Cancer Cells

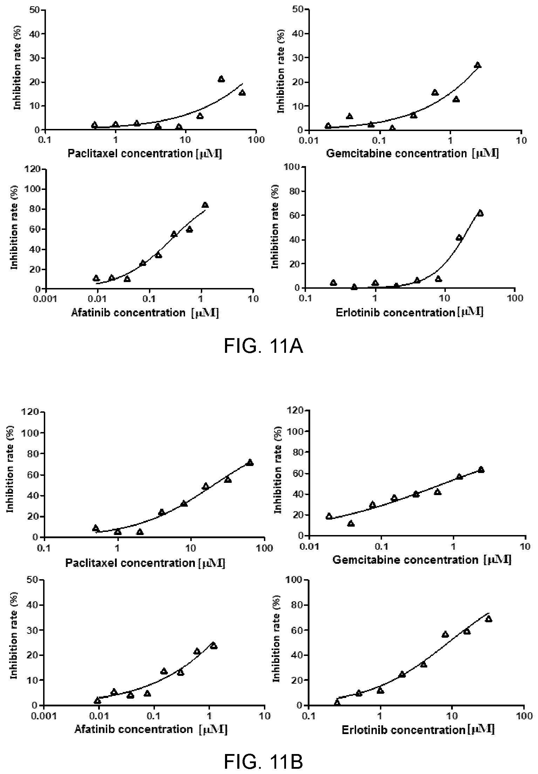

Taking a surgical resection sample from a lung cancer patient as an example, it is verified that the lung cancer cells cultured from the patient-derived lung cancer samples can be used to test the sensitivity of the tumor cells of the patient to different drugs.
1. Plating of primary lung cancer cells: Cell suspensions of isolated lung cancer cells (No. B25 and No. B26) obtained according to the method of Example 1, were inoculated in a 12-well plate which was coated with Matrigel^{®} at a density of 4×10⁴ cells/cm². 2 ml of the prepared culture medium FLM for primary lung cancer epithelial cells was added to the 12-well plate, which was then placed in a 37°C, 5% CO₂ incubator (purchased from Thermo Fisher) for culture. When the cells in the culture plate grew to cover about 80% of the bottom area, the medium supernatant in the 12-well plate was discarded and 500 µl of 0.05% trypsin (purchased from Gibco) was added for cell digestion; the cells were incubated at 37°C for 10 minutes until the cells were completely digested as observed under a microscope (EVOS M500, Invitrogen); then the digestion was terminated by using 1 ml of DMEM/F12 culture solution containing 5% (v/v) fetal bovine serum, 100 U/ml penicillin, and 100 µg/ml streptomycin; the resultant was collected into a 15 ml centrifuge tube and centrifuged at 1500 rpm for 4 minutes, and then the supernatant was discarded. The centrifuged cell pellet was resuspended in the culture medium FLM and the cells were counted with a flow imaging counter (JIMBIO FIL, Jiangsu Jimbio Technology Co., Ltd.) to get the total number of cells, which were 880,000 and 680,000, respectively. The cells were incubated into a 384-well plate at a density of 2,000-4,000 cells/well, and the cells were adhered overnight.
2. Drug gradient experiments:
   (1) The drug storage plates were prepared by gradient dilution method: 10 µl drug stock solutions (the drug stock solutions were prepared to have a concentration of 2 times of the maximum plasma concentration Cₘₐₓ of the drug in the human body) to be tested were respectively pipetted, and added into 0.5 ml EP tubes containing 20 µl of DMSO; 10 µl of solutions from the above EP tubes were pipetted again into second 0.5 ml EP tubes loaded with 20 µl of DMSO, that is, diluting the drugs in a ratio of 1:3. The above method was repeated for gradually dilution and 8 concentrations required for dosing were obtained. Different concentrations of drugs were added to a 384-well drug storage plate. Equal volume of DMSO was added to each well of the solvent control group as a control. In this example, the drugs to be tested were Paclitaxel (purchased from MCE), Gemcitabine (purchased from MCE), Afatinib (purchased from MCE), and Erlotinib (purchased from MCE).
   (2) Using a high-throughput automated workstation (JANUS, Perkin Elmer), different concentrations of drugs and solvent controls in the 384-well drug storage plates were added to 384-well cell culture plates plated with the lung cancer cells. The drug groups and the solvent control groups were each arranged with 3 duplicate wells. The volume of drugs added to each well was 100 nL.
   (3) Test of cell viability: 72 hours after administration, Cell Titer-Glo assay kit (purchased from Promega) was used to detect the chemiluminescence value of the cultured cells after drug administration. The magnitude of the chemiluminescence value reflects the cell viability and the effect of the drug on the cell viability. 10 µl of the prepared Cell Titer-Glo detection solution was added to each well, and a microplate reader (Envision, Perkin Elmer) was used to detect the chemiluminescence value after mixing.
   (4) Test of cell viability: According to the formula, Cell survival rate (%) = Chemiluminescence value of drug well / Chemiluminescence value of control well × 100%, the cell survival rates of cells treated with different drugs were calculated. Graphs were made by using Graphpad Prism software and the half-inhibition rates IC₅₀ were calculated.
   (5) The drug sensitivity testing results were shown in Figures 11A and 11B.

Figures 11A and 11B respectively represent the sensitivity of the lung cancer cells cultured from surgically resected cancer tissue samples of two different lung cancer patients (Sample No. B25 and Sample No. B26) to two chemotherapeutic drugs Paclitaxel and Gemcitabine, and to two targeted drugs Afatinib and Erlotinib. Specifically, Figure 11A shows the sensitivity results of lung cancer cells cultured from Sample No. B25 on four drugs; Figure 11B shows the sensitivity results of lung cancer cells cultured from Sample No. B26 on four drugs. The results show that the cells from the same patient have different sensitivities to different drugs, and the cells from different patients also have different sensitivities to the same drug.

### Industrial applicability

The invention provides a culture medium and a culture method for culturing primary lung cancer epithelial cells. The cultured lung cancer epithelial cells can be used for the efficacy evaluating and screening of drugs. Therefore, the invention is applicable in the industry.

Although the invention has been described in details herein, the invention is not limited thereto, and those skilled in the art can make modification according to the principle of the invention. Therefore, all modifications made according to the principle of the invention should be interpreted as falling within the protection scope of the invention.

## Claims

1. A primary cell culture medium for culturing primary lung cancer epithelial cells, **characterized in that**:
comprising an MST1/2 kinase inhibitor; a ROCK kinase inhibitor selected from at least one of Y27632, Fasudil, and H-1152; a fibroblast growth factor; at least one additive selected from a B27 additive and an N2 additive; an epidermal growth factor; transferrin; gastrin; and a TGFβ type I receptor inhibitor selected from at least one of A83-01, SB431542, Repsox, SB505124, SB525334, SD208, LY36494, and SJN2511,
wherein the MST1/2 kinase inhibitor comprises a compound of Formula (I) or a pharmaceutically acceptable salt, or a solvate thereof, 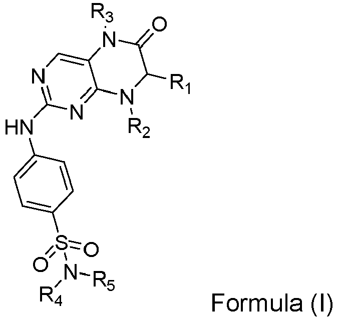 wherein,
R₁ is selected from C1-C6 alkyl, C3-C6 cycloalkyl, C4-C8 cycloalkylalkyl, C2-C6 spirocycloalkyl, and aryl optionally substituted with 1-2 independent R₆, aryl C1-C6 alkyl optionally substituted with 1-2 independent R₆ and heteroaryl optionally substituted with 1-2 independent R₆;
R₂ and R₃ are each independently selected from C1-C6 alkyl;
R₄ and R₅ are each independently selected from hydrogen, C1-C6 alkyl, C3-C6 cycloalkyl, C4-C8 cycloalkylalkyl, hydroxyl C1-C6 alkyl, C1-C6 haloalkyl, C1-C6 alkylamino C1-C6 alkyl, C1-C6 alkoxy C1-C6 alkyl, and C3-C6 heterocyclyl C1-C6 alkyl;
R₆ is selected from halogen, C1-C6 alkyl, C1-C6 alkoxy, and C1-C6 haloalkyl.

2. The primary cell culture medium of claim 1, wherein,
R₁ is selected from C1-C6 alkyl, C3-C6 cycloalkyl, C4-C8 cycloalkylalkyl, C2-C6 spirocycloalkyl, and phenyl optionally substituted with 1-2 independent R₆, naphthyl optionally substituted with 1-2 independent R₆, phenylmethyl optionally substituted with 1-2 independent R₆ and thienyl optionally substituted with 1-2 independent R₆;
R₂ and R₃ are each independently selected from C1-C3 alkyl;
R₄ and R₅ are each independently selected from hydrogen, C1-C6 alkyl, C3-C6 cycloalkyl, C4-C8 cycloalkylalkyl, hydroxyl C1-C6 alkyl, C1-C6 haloalkyl, C1-C6 alkylamino C1-C6 alkyl, C1-C6 alkoxy C1-C6 alkyl, piperidyl C1-C6 alkyl, and tetrahydropyranyl C1-C6 alkyl;
R₆ is selected from halogen, C1-C6 alkyl, C1-C6 alkoxy, and C1-C6 haloalkyl.

3. The primary cell culture medium of claim 1, wherein the MST1/2 kinase inhibitor comprises a compound of Formula (Ia) or a pharmaceutically acceptable salt, or a solvate thereof, 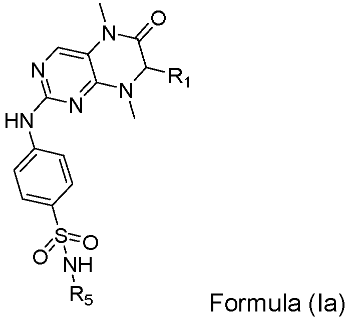 wherein,
R₁ is selected from C1-C6 alkyl, phenyl optionally substituted with 1-2 independent R₆, thienyl optionally substituted with 1-2 independent R₆, and phenylmethyl optionally substituted with 1-2 independent R₆;
R₅ is selected from hydrogen, C1-C6 alkyl, and C3-C6 cycloalkyl;
R₆ is independently selected from halogen, C1-C6 alkyl, and C1-C6 haloalkyl.

4. The primary cell culture medium of claim 3, wherein
R₁ is phenyl optionally substituted with 1-2 independent R₆;
R₅ is hydrogen;
R₆ is fluoro, methyl or trifluoromethyl.

5. The primary cell culture medium of claim 1, wherein the MST1/2 kinase inhibitor is at least one selected from the following compounds or a pharmaceutically acceptable salt thereof:
| | | | |
|---|---|---|---|
| 1 | 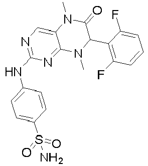 | 2 | 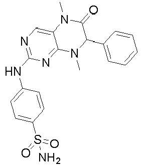 |
| 3 | 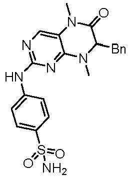 | 4 | 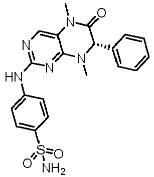 |
| 5 | 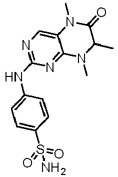 | 6 | 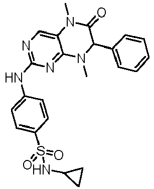 |
| 7 | 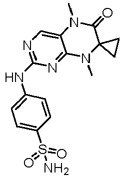 | 8 | 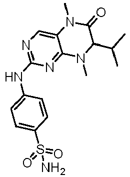 |
| 9 | 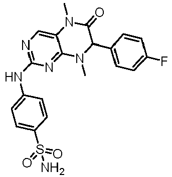 | 10 | 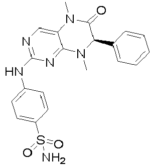 |
| 11 | 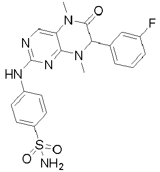 | 12 | 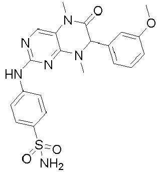 |
| 13 | 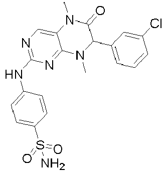 | 14 | 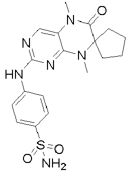 |
| 15 | 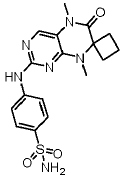 | 16 | 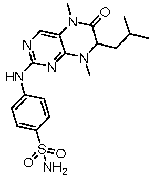 |
| 17 | 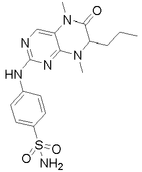 | 18 | 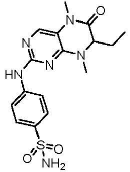 |
| 19 | 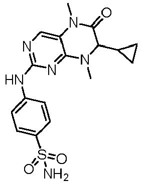 | 20 | 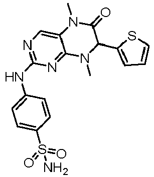 |
| 21 | 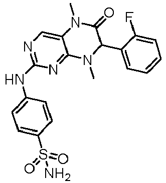 | 22 | 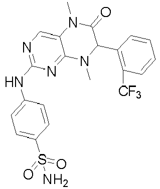 |
| 23 | 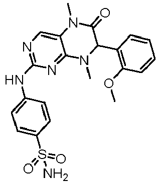 | 24 | 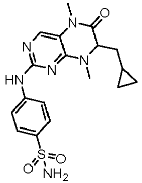 |
| 25 | 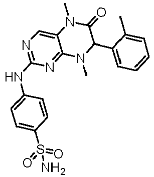 | 26 | 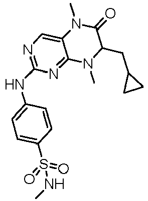 |
| 27 | 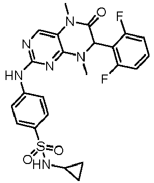 | 28 | 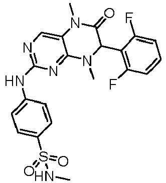 |
| 29 | 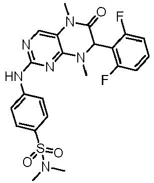 | 30 | 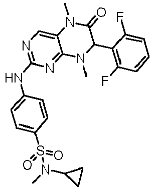 |
| 31 | 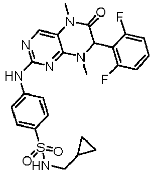 | 32 | 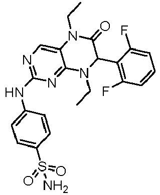 |
| 33 | 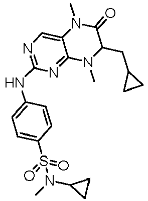 | 34 | 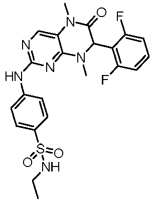 |
| 35 | 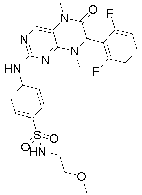 | 36 | 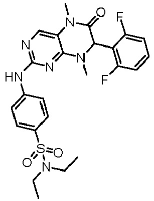 |
| 37 | 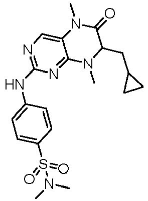 | 38 | 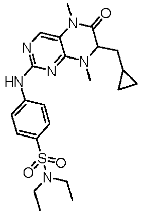 |
| 39 | 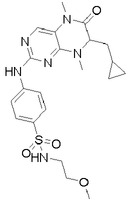 | 40 | 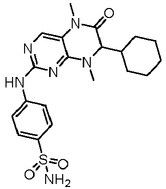 |
| 41 | 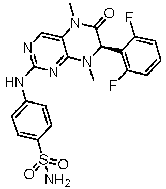 | 42 | 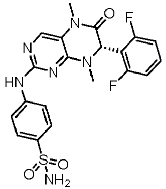 |
| 43 | 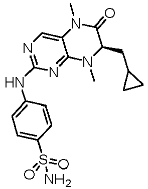 | 44 | 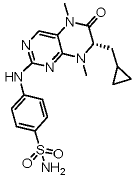 |
| 45 | 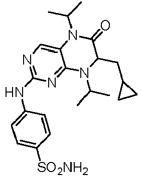 | 46 | 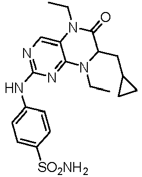 |
| 47 | 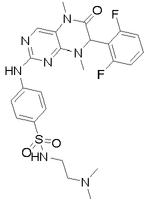 | 48 | 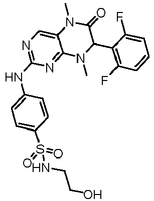 |
| 49 | 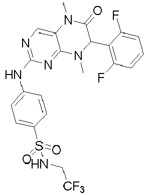 | 50 | 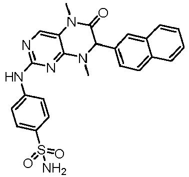 |
| 51 | 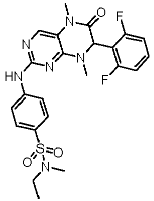 | 52 | 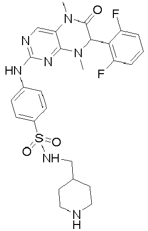 |
| 53 | 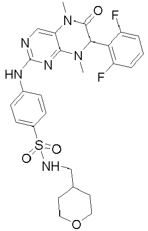 | 54 | 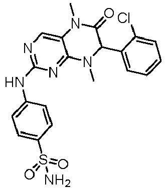 |
| 55 | 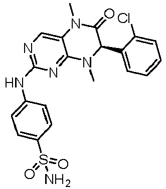 | 56 | 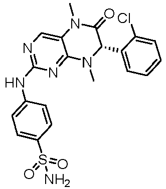 |
| 57 | 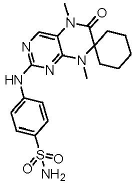 | 58 | 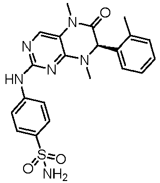 |
| 59 | 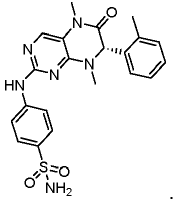 | | |

6. The primary cell culture medium of any one of claims 1-5, **characterized in that**:
the amount of the MST1/2 kinase inhibitor in the culture medium is 2.5-15 µM, preferably 2.5-10 µM.

7. The primary cell culture medium of any one of claims 1-6, **characterized in that**, the primary cell culture medium satisfies any one or more or all of the following conditions:
the amount of the ROCK kinase inhibitor in the culture medium is 2.5-18 µM, preferably 5-15 µM;
the amount of the fibroblast growth factor is 2.5-80 ng/ml, preferably 5-40 ng/ml;
the volume concentration of the B27 additive or the N2 additive in the culture medium is 1:25-1:800, preferably 1:25-1:200;
the amount of the epidermal growth factor is 2.5-80 ng/ml, preferably 10-40 ng/ml;
the amount of transferrin is 2.5-80 ng/ml, preferably 5-80 ng/ml;
the amount of gastrin is 2.5-80 ng/ml, preferably 5-40 ng/ml;
the amount of the TGFβ type I receptor inhibitor is 62.5-800 nM, preferably 125-500 nM.

8. The primary cell culture medium of any one of claims 1-7, **characterized in that**, the primary cell culture medium satisfies any one or more or all of the following conditions:
the MST1/2 kinase inhibitor is Compound 1;
the ROCK kinase inhibitor is Y27632;
the TGFβ type I receptor inhibitor is A83-01.

9. The primary cell culture medium of any one of claims 1-8, **characterized in that**, further comprising:
an initial medium selected from the group consisting of DMEM/F12, DMEM, F12 or RPMI-1640; and
one or more antibiotics selected from the group consisting of streptomycin/penicillin, amphotericin B and Primocin.

10. The primary cell culture medium of any one of claims 1-9, **characterized in that**,
being free of serum, bovine pituitary extract, Wnt agonists, R-spondin family proteins, BMP inhibitors, nicotinamide, or N-acetylcysteine.

11. The primary cell culture medium of any one of claims 1-10, **characterized in that**:
the primary lung cancer epithelial cells are selected from the group consisting of lung cancer cells, normal lung cancer epithelial cells, and lung cancer epithelial stem cells.

12. A method for culturing primary lung cancer epithelial cells, **characterized in that**, comprising the following steps:
(1) preparing the primary cell culture medium according to any one of claims 1-11;
(2) coating a culture vessel with extracellular matrix gel dilution;
(3) inoculating primary lung cancer epithelial cells isolated from lung cancer tissues in the culture vessel which is coated with extracellular matrix gel, and culturing by using the primary cell culture medium of step (1).

13. A method for evaluating or screening a drug for treating lung cancer, **characterized in that**, comprising the following steps:
(1) culturing lung cancer epithelial cells by the culturing method of claim 12;
(2) selecting the drug to be tested and diluting into different drug concentration gradients;
(3) adding the drug which has been diluted to gradients to the lung cancer epithelial cells obtained in step (1), and detecting the cell viability.
